# EUROPÄISCHE PATENTSCHRIFT

(11) **EP 1 289 514 B1**
(45) Veröffentlichungstag und Bekanntmachung des Hinweises auf die Patenterteilung: **03.11.2004**
(21) Anmeldenummer: 01940450.8
(22) Anmeldetag: 10.05.2001
(51) Int. Cl.: A61K 31/137, A61K 31/4453, A61K 31/402, A61K 31/5375, A61P 25/00

(54) **VERWENDUNG SUBSTITUIERTER 1-AMINO-5-PHENYLPENTAN-3-OL- UND/ODER 1-AMINO-6-PHENYLHEXAN-3-OL-VERBINDUNGEN ALS ARZNEIMITTEL**
USE OF SUBSTITUTED 1-AMINO-5-PHENYLPENTANE-3-OL AND/OR 1-AMINO-6-PHENYLHEXANE-3-OL COMPOUNDS AS MEDICAMENTS
UTILISATION COMME MEDICAMENTS DE COMPOSES 1-AMINO-5-PHENYLPENTAN-3-OL ET/OU 1-AMINO-6-PHENYLHEXAN-3-OL SUBSTITUES

(30) Priorität: 22.05.2000 DE 10025238
(43) Veröffentlichungstag der Anmeldung: 12.03.2003
(73) Patentinhaber: Grünenthal GmbH, 52078 Aachen (DE)
(72) Erfinder: SUNDERMANN, Bernd, 52066 Aachen (DE); ENGLBERGER, Werner, 52223 Stolberg (DE); CHIZH, Boris, Cambridge CB2 4UW (GB)
(74) Vertreter: Kutzenberger, Helga, Dr.
(86) Internationale Anmeldenummer: PCT/EP2001/005349
(87) Internationale Veröffentlichungsnummer: WO 2001/089505

(56) Entgegenhaltungen:
- EP-A- 1 043 307
- WO-A-92/05169
- IHL ET AL.: "Zur Nootropikabewertung für die Praxis" NERVENARZT, Bd. 68, Nr. 11, 1997, Seiten 853-861, XP001013225

## Beschreibung

Die Erfindung betrifft die Verwendung wenigstens einer substituierten 1-Amino-5-phenylpentan-3-ol- und/oder 1-Amino-6-phenylhexan-3-ol-Verbindung und/oder eines ihrer Enantiomeren und/oder eines ihrer Diastereomeren und/oder eines der entsprechenden physiologisch verträglichen Salze zur Herstellung eines Arzneimittels mit N-Methyl-D-Aspartat-(NMDA)-antagonistischer Wirkung.

Kenntnisse über die physiologische Bedeutung von lonenkanal-selektiven Substanzen sind durch die Entwicklung der patch-clamp-Technik gewonnen worden. Von besonderer Bedeutung ist der NMDA-Ionenkanal, über den ein wesentlicher Teil der Kommunikation von Synapsen abläuft. Durch diesen Ionenkanal wird der Austausch von Calciumionen zwischen einer neuronalen Zelle und ihrer Umgebung gesteuert. Die Wirkung von NMDA-Antagonisten auf den Einstrom von Calciumionen in das Zellinnere konnte mittels patch-clamp-Technik nachgewiesen werden.

Im nichtaktivierten Zustand sind die NMDA-Ionenkanäle jeweils durch einzelne Magnesiumionen verschlossen, die sich im Inneren des Kanals befinden und diesen aufgrund ihrer Größe nicht passieren können. Im aktivierten Zustand können die kleineren Calcium- und Natriumionen den Kanal passieren. Die (+)-MK801-Bindungsstelle des NMDA-Ionenkanals (ionotroper NMDA-Rezeptor) befindet sich ebenfalls im Inneren dieses Membranproteins. Substanzen mit NMDA-antagonistischer Wirkung, wie Phencyclidin (PCP), Ketamin oder MK801, besetzen diese Bindungsstelle (sogenannte "Channelblocker") und verschließen daher den betreffenden NMDA-Ionenkanal.

NMDA-lonenkanäle spielen in vielen physiologischen und pathophysiologischen Prozessen eine wichtige Rolle, wie z.B bei der Epilepsie, der Schizophrenie, neurodegenerativen Erkrankungen, insbesondere bei Morbus Alzheimer, Morbus Huntington und Morbus Parkinson, cerebralen Ischämien und Infarkten, Psychosen bedingt durch erhöhten Aminosäurespiegel, Hirnödemen,
Unterversorgungszuständen des zentralen Nervensystems, insbesondere bei Hypoxien und Anoxien, der AIDS-Demens, der Encephalomyelitis, dem Tourette-Syndrom, der perinatalen Asphyxie und bei Tinnitus, wie beispielsweise in EP 0 869 122 oder US 5,216,003 beschrieben.

Aufgabe der vorliegenden Erfindung war es daher, Arzneimittel zur Verfügung zu stellen, die NMDA-antagonistische Wirkung zeigen und damit zur Prophylaxe von Schlaganfällen und/oder Behandlung von Epilepsie und/oder Schizophrenie und/oder neurodegenerativen Erkrankungen, insbesondere Morbus Alzheimer, Morbus Huntington oder Morbus Parkinson, und/oder cerebralen Ischämien und/oder cerebralen Infarkten und/oder Psychosen bedingt durch erhöhten Aminosäurespiegel und/oder Hirnödemen und/oder Unterversorgungszuständen des zentralen Nervensystems, insbesondere Hypoxie und/oder Anoxie, und/oder AIDS-Demens und/oder Encephalomyelitis und/oder Tourette-Syndrom und/oder perinataler Asphyxie und/oder Tinnitus geeignet sind.

Überraschenderweise wurde gefunden, daß substituierte 1-Amino-5-phenylpentan-3-ol- und 1-Amino-6-phenylhexan-3-ol-Verbindungen der nachstehenden allgemeinen Formel I, deren Enantiomeren, Diastereomeren und deren physiologisch verträgliche Salze eine ausgeprägte NMDA antagonistische Wirkung zeigen und damit zur Beeinflussung der vorstehend genannten physiologischen und pathophysiologischen Prozesse sehr gut geeignet sind.

Gegenstand der vorliegenden Erfindung ist daher die Verwendung wenigstens einer substituierten 1-Amino-5-phenylpentan-3-ol- und/oder 1-Amino-6-phenylhexan-3-ol-Verbindung der allgemeinen Formel I, 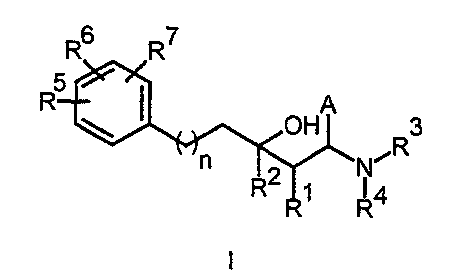 worin n = 1 oder 2 ist,
der Rest A einen gegebenenfalls substituierten Aryl- oder Heteroarylrest bedeutet,
die Reste R¹ und R², gleich oder verschieden, für einen C₁₋₆-Alkyl-, vorzugsweise einen C₁₋₃-Alkylrest stehen oder die Reste R¹ und R² zusammen eine (CH₂)₂₋₆-Kette bilden, die auch phenylsubstituiert oder benzokondensiert sein kann,
die Reste R³ und R⁴, gleich oder verschieden, für einen C₁₋₆-Alkyl-, vorzugsweise einen C₁₋₃-Alkyl-, einen gegebenenfalls substituierten Aryl-, oder für einen über eine C₁₋₃-Alkylen-Gruppe gebundenen, ggf. substituierten Aryl-Rest stehen oder die Reste R³ und R⁴ zusammen (CH₂)₃₋₆ oder CH₂CH₂OCH₂CH₂ bedeuten,
die Reste R⁵, R⁶, R⁷, gleich oder verschieden, H, F, Cl, Br, I, CF₃, OR⁸, SO₂CH₃, SO₂CF₃, Phenyl, CN, NO₂ oder einen C₁₋₆-Alkyl-, vorzugsweise einen C₁₋₃-Alkyl-Rest bedeuten,
der Rest R⁸ für H, einen C₁₋₆-Alkyl-, vorzugsweise einen C₁₋₃-Alkyl-, einen gegebenenfalls substituierten Aryl-, einen gegebenenfalls substituierten Heteroaryl- oder für einen über eine C₁₋₃-Alkylen-Gruppe gebundenen, gegebenenfalls substituierten Aryl- oder Heteroaryl-Rest steht,
und/oder eines ihrer Enantiomeren und/oder eines ihrer Diastereomeren und/oder eines entsprechenden physiologisch verträglichen Salzes zur Herstellung eines Arzneimittels mit NMDA antagonistischer Wirkung gemäß Anspruch 1.

Als physiologisch verträgliches Salz der Verbindungen der allgemeinen Formel I und/oder ihrer Enantiomeren und/oder ihrer Diastereomeren kann bevorzugt das Hydrochlorid, Hydrobromid, Sulfat, Sulfonat, Phosphat, Tartrat, Embonat, Formiat, Acetat, Propionat, Benzoat, Oxalat, Succinat, Citrat, Glutamat, Fumarat, Aspartat, Glutarat, Stearat, Butyrat, Malonat, Lactat, Mesylat oder ein Gemisch aus wenigstens zwei dieser Salze eingesetzt werden.

Unter Alkyl-Resten werden verzweigte, unverzweigte und cyclische Kohlenwasserstoff-Reste verstanden, die auch mindestens einfach, vorzugsweise mit einem Halogen- und/oder einem Hydroxyl-Rest, besonders bevorzugt mit Fluor und/oder einem Hydroxyl-Rest substituiert sein können. Enthalten diese Alkyl-Reste mehr als einen Substituenten, so können diese Substituenten gleich oder verschieden sein. Vorzugsweise sind die Alkyl-Reste Methyl, Ethyl, Propyl, 1-Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, Cyclopropylmethyl, 2-Methylcyclopropyl, Cyclopentyl, Cyclohexyl, CHF₂, CF₃ oder CH₂OH.

Unter einem Aryl-Rest werden auch mindestens einfach mit einem OR⁸-, einem Halogen-, vorzugsweise F- und/oder Cl-, einem CN-, einem NO₂-, einem C₁₋₆-Alkyl- oder einem Phenylrest substituierte Phenyl- oder Naphtylreste verstanden, wobei der Rest R⁸ die Bedeutung gemäß der allgemeinen Formel I hat. Die Phenylreste können auch mit weiteren Ringen kondensiert sein.

Unter einem Heteroaryl-Rest werden auch 5- oder 6-gliedrige, ungesättigte, gegebenenfalls ein System von ankondensierten Aryl-Resten enthaltende, heterocyclische Verbindungen verstanden, die wenigstens ein Heteroatom, vorzugsweise Stickstoff, Sauerstoff oder Schwefel, besonders bevorzugt Stickstoff oder Sauerstoff enthalten und die ggf. auch mindestens einfach mit einem OR⁸-, einem Halogen-, vorzugsweise F- und/oder Cl-, einem CN-, einem NO₂-, einem C₁₋₆-Alkyl-, oder Phenyl-Rest substituiert sein können, wobei der Rest R⁸ die Bedeutung gemäß der allgemeinen Formel I hat. Bevorzugte, ggf. substituierte Heteroaryl-Reste sind Furan, Thiophen, Pyrrol, Pyridin, Pyrimidin, Chinolin, Isochinolin, Phthalazin oder Chinazolin.

In einer bevorzugten Ausführungsform der vorliegenden Erfindung wird wenigstens eine Verbindung der allgemeinen Formel I eingesetzt, in der die Reste R¹ und R² zusammen eine (CH₂)₂₋₆-Kette bilden, die auch phenylsubstituiert oder benzokondensiert sein kann, und die Reste R³ bis R⁸ und A die Bedeutung gemäß der allgemeinen Formel I haben.

Ebenfalls bevorzugt ist die Verwendung wenigstens einer Verbindung der allgemeinen Formel I, in der A einen unsubstituierten oder substituierten Phenyl-, Thiophenyl- oder Furyl-Rest bedeutet und die Reste R¹ bis R⁸ die Bedeutung gemäß der allgemeinen Formel I haben.

Bevorzugt ist auch die Verwendung wenigstens einer Verbindung der allgemeinen Formel I, in der die Reste R⁵ bis R⁷, gleich oder verschieden, H, einen Halogen- oder einen CF₃-Rest bedeuten und die Reste R¹ bis R⁴, R⁸ und A die Bedeutung gemäß der allgemeinen Formel I haben.

Bevorzugt ist auch die Verwendung wenigstens einer Verbindung der allgemeinen Formel I, in der der Phenylring der allgemeinen Formel I ein- oder zweifach in ortho-Position substituiert ist und die Reste R¹ bis R⁸ sowie A die Bedeutung gemäß der allgemeinen Formel I haben.

Bevorzugt ist weiterhin auch die Verwendung wenigstens einer Verbindung der allgemeinen Formel I, in der A einen Phenylring darstellt, der ein- oder zweifach in ortho-Position substituiert ist, und die Reste R¹ bis R⁸ die Bedeutung gemäß der allgemeinen Formel I haben.

In einer besonders bevorzugten Ausführungsform der vorliegenden Erfindung wird wenigstens eine Verbindung der allgemeinen Formel I, in der R¹ und R² zusammen einen Cyclohexylring bilden, der auch phenylsubstituiert oder benzokondensiert sein kann, A einen unsubstituierten oder substituierten Phenyl-, Thiophenyl- oder Furyl-Rest bedeutet und die Reste R³ bis R⁸ die Bedeutung gemäß der allgemeinen Formel I haben.

Besonders bevorzugt ist auch die Verwendung wenigstens einer Verbindung der allgemeinen Formel I, in der A einen Phenylring darstellt, der ein- oder zweifach in ortho-Position substituiert ist, und in der der Phenylring der allgemeinen Formel I ein- oder zweifach in ortho-Position substituiert ist und die Reste R¹ bis R⁸ die Bedeutung gemäß der allgemeinen Formel I haben.

Ganz besonders bevorzugt ist die Verwendung wenigstens einer der nachfolgenden Verbindungen der allgemeinen Formel I:
2-(Dimethylaminophenylmethyl)-1-phenethylcyclohexanol oder das entsprechende Hydrochlorid,
2-[(2-Chlorphenyl)dimethylaminomethyl]-1-phenethylcyclohexanol oder das entsprechende Hydrochlorid,
2-[(2-Bromphenyl)dimethylaminomethyl]-1-phenethylcyclohexanol oder das entsprechende Hydrochlorid,
2-[Dimethylamino-(3-methoxyphenyl)methyl]-1-phenethylcyclohexanol oder das entsprechende Hydrochlorid,
2-(Dimethylamino-o-tolylmethyl)-1-phenethylcyclohexanol oder das entsprechende Hydrochlorid,
2-(Dimethylaminophenylmethyl)-1-(3-phenylpropyl)cyclohexanol oder das entsprechende Hydrochlorid,
2-(Dimethylaminophenylmethyl)-1-[2-(2-fluorphenyl)ethyl]cyclohexanol oder das entsprechende Hydrochlorid,
2-[Dimethylamino-(2-fluorphenyl)methyl]-1-phenethylcyclohexanol oder das entsprechende Hydrochlorid,
2-[(2-Chlorphenyl)dimethylaminomethyl]-1-[2-(4-fluorphenyl)ethyl]cyclohexanol oder das entsprechende Hydrochlorid,
2-[(2-Chlorphenyl)dimethylaminomethyl]-1-[2-(2-fluorphenyl)ethyl]cyclohexanol oder das entsprechende Hydrochlorid,
2-[(2-Chlorphenyl)dimethylaminomethyl]-1-[2-(3-fluorphenyl)ethyl]cyclohexanol oder das entsprechende Hydrochlorid,
1-[2-(2-Chlorphenyl)ethyl]-2-(dimethylaminophenylmethyl)cyclohexanol oder das entsprechende Hydrochlorid,
1-[2-(3-Chlorphenyl)ethyl]-2-(dimethylaminophenylmethyl)cyclohexanol oder das entsprechende Hydrochlorid,
1-Phenethyl-2-(phenylpiperidin-1-yl-methyl)cyclohexanol oder das entsprechende Hydrochlorid,
1-[2-(2-Chlorphenyl)ethyl]-2-(dimethylamino-o-tolylmethyl)cyclohexanol oder das entsprechende Hydrochlorid,
1-[2-(3-Chlorphenyl)ethyl]-2-(dimethylamino-o-tolylmethyl)cyclohexanol oder das entsprechende Hydrochlorid,
2-[Dimethylamino-(2-fluorphenyl)methyl]-1-[2-(4-fluorphenyl)ethyl]cyclohexanol oder das entsprechende Hydrochlorid,
2-(Dimethylamino-o-tolylmethyl)-1-[2-(4-fluorphenyl)ethyl]cyclohexanol oder das entsprechende Hydrochlorid,
1-[2-(2-Chlorphenyl)ethyl]-2-[dimethylamino-(2-fluorphenyl)methyl]cyclohexanol oder das entsprechende Hydrochlorid,
1-[2-(2-Chlorphenyl)ethyl]-2-(dimethylamino-m-tolylmethyl)cyclohexanol oder das entsprechende Hydrochlorid,
1-[2-(3-Chlorphenyl)ethyl]-2-[dimethylamino-(2-fluorphenyl)methyl]cyclohexanol oder das entsprechende Hydrochlorid,
1-[2-(2-Chlorphenyl)ethyl]-2-[dimethylamino-(3-fluorphenyl)methyl]cylohexanol oder das entsprechende Hydrochlorid,
1-[2-(2-Chlorphenyl)ethyl]-2-(pyrrolidin-1-yl-o-tolylmethyl)cyclohexanol oder das entsprechende Hydrochlorid,
1-[2-(2-Chlorphenyl)ethyl]-2-(morpholin-4-yl-o-tolylmethyl)cyclohexanol oder das entsprechende Hydrochlorid,
1-[2-(2-Chlorphenyl)ethyl]-2-(piperidin-1-yl-o-tolylmethyl)cyclohexanol oder das entsprechende Hydrochlorid,
1-[2-(3-Chlorphenyl)ethyl]-2-(dimethylamino-o-tolylmethyl)cyclohexanol oder das entsprechende Hydrochlorid,
2-[Dimethylamino-(2-trifluormethylphenyl)methyl]-1-phenethylcyclohexanol oder das entsprechende Hydrochlorid,
2-[Dimethylamino-(2-methoxyphenyl)methyl]-1-[2-(2-fluorphenyl)ethyl]cyclohexanol oder das entsprechende Hydrochlorid,
2-[Dimethylamino-(2-fluorphenyl)methyl]-1-[2-(2-fluorphenyl)ethyl]cyclohexanol oder das entsprechende Hydrochlorid,
2-(Dimethylamino-o-tolylmethyl)-1-[2-(2-fluorphenyl)ethyl]cyclohexanol oder das entsprechende Hydrochlorid,
1-[2-(2-Fluorphenyl)ethyl]-2-(pyrrolidin-1-yl-o-tolylmethyl)cyclohexanol oder das entsprechende Hydrochlorid,
2-[(2-Bromphenyl)dimethylaminomethyl]-1-[2-(2-chlorphenyl)ethyl]cyclohexanol oder das entsprechende Hydrochlorid,
2-[(2-Chlorphenyl)dimethylaminomethyl]-1-[2-(2-chlorphenyl)ethyl]cyclohexanol oder das entsprechende Hydrochlorid,
2-[(2-Bromphenyl)dimethylaminomethyl]-1-[2-(2-fluorphenyl)ethyl]cyclohexanol oder das entsprechende Hydrochlorid,
2-[(2-Chlorphenyl)dimethylaminomethyl]-1-(2-o-tolylethyl)cyclohexanol oder das entsprechende Hydrochlorid,
2-[(2-Chlorphenyl)dimethylaminomethyl]-1-(3-phenylpropyl)cyclohexanol oder das entsprechende Hydrochlorid,
2-(Dimethylaminophenylmethyl)-1-(2-o-tolylethyl)cyclohexanol oder das entsprechende Hydrochlorid,
2-[(2-Bromphenyl)dimethylaminomethyl]-1-(2-o-tolylethyl)cyclohexanol oder das entsprechende Hydrochlorid,
2-(Dimethylamino-o-tolylmethyl)-1-(2-o-tolylethyl)cyclohexanol oder das entsprechende Hydrochlorid,
2-[(2-Bromphenyl)dimethylaminomethyl]-1-(3-phenylpropyl)cyclohexanol oder das entsprechende Hydrochlorid,
2-[(2-Chlor4-fluorphenyl)dimethylaminomethyl]-1-[2-(2-fluorphenyl)ethyl]cyclohexanol oder das entsprechende Hydrochlorid,
2-[(2-Chlor4-fluorphenyl)dimethylaminomethyl]-1-[2-(2-chlorphenyl)ethyl]cyclohexanol oder das entsprechende Hydrochlorid,
2-[Dimethylamino-(2-fluorphenyl)methyl]-1-(2-o-tolylethyl)cyclohexanol oder das entsprechende Hydrochlorid,
2-[Dimethylamino-(2-fluorphenyl)methyl]-1-(3-phenylpropyl)cyclohexanol oder das entsprechende Hydrochlorid,
2-(Dimethylamino-o-tolylmethyl)1-(3-phenylpropyl)cyclohexanol oder das entsprechende Hydrochlorid,
1-[2-(2-Chlorphenyl)ethyl]-2-[dimethylamino-(2-trifluomethylphenyl)methyl]-cyclohexanol oder das entsprechende Hydrochlorid,
   oder
2-[Dimethylamino-(2-trifluormethylphenyl)methyl]-1-[2-(2-fluorphenyl)ethyl]-cyclohexanol oder das entsprechende Hydrochlorid.

Ein weiterer Gegenstand der Erfindung ist auch die Verwendung wenigstens einer substituierten 1-Amino-5-phenylpentan-3-ol- und/oder 1-Amino-6-phenylhexan-3-ol-Verbindung der allgemeinen Formel I zur Herstellung eines Arzneimittels zur Prophylaxe von Schlaganfällen und/oder zur Behandlung von Epilepsie und/oder Schizophrenie und/oder neurodegenerativen Erkrankungen, insbesondere Morbus Alzheimer und/oder Morbus Huntington und/oder Morbus Parkinson, und/oder von cerebralen Ischämien und/oder cerebralen Infarkten und/oder Psychosen bedingt durch erhöhten Aminosäurespiegel und/oder Himödemen und/oder Unterversorgungszuständen des zentralen Nervensystems, insbesondere Hypoxie und/oder Anoxie, und/oder AIDS-Demens und/oder Encephalomyelitis und/oder Tourette-Syndrom und/oder perinataler Asphyxie und/oder Tinnitus.

Das oben genannte Arzneimittel kann auch ein Gemisch aus Enantiomeren wenigstens einer Verbindung der allgemeinen Formel I enthalten, wobei die Enantiomeren in diesem Gemisch nicht in äquimolaren Mengen vorliegen. Vorzugsweise beträgt der relative Anteil eines der Enantiomeren an einem solchen Enantiomerengemisch 5 bis 45 Massenprozent.

Zur Zubereitung der Arzneimittel können neben wenigstens einer Verbindung der allgemeinen Formel I weitere Hilfsstoffe, wie Trägermaterialien, Füllstoffe, Lösungsmittel, Verdünnungsmittel, Farbstoffe und/oder Bindemittel eingesetzt werden. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Dem Fachmann sind die für die jeweilige Applikationsform geeigneten Hilfsstoffe sowie deren Mengen bekannt. Für die orale Applikation eignen sich Arzneimittel in Form von Tabletten, Kautabletten, Kaugummis, Dragees, Kapseln, Granulaten, Tropfen, Säften und Sirupen, für die parenterale, topische und inhalative Applikation eignen sich vorzugsweise Lösungen, Suspensionen, Emulsionen, leicht rekonstituierbare Trockenzubereitungen, Spheroide, Sprays, Suppositorien oder Pflaster, wie z.B. transdermale therapeutische Systeme. Zur buccalen Applikation eignet sich vorzugsweise ein transmucales therapeutisches System. Die Verbindungen der allgemeinen Formel I in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die Verbindungen der allgemeinen Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,5 bis 50 mg pro kg Körpergewicht des Patienten wenigstens einer Verbindung der allgemeinen Formel I appliziert.

Die substituierten 1-Amino-5-phenylpentan-3-ol- und 1-Amino-6-phenylhexan-3-ol-Verbindungen der allgemeinen Formel I können wie nachstehend beschrieben hergestellt werden. Die Reste R¹ bis R⁷ sowie A haben in den nachstehenden allgemeinen Formeln II bis XI jeweils die Bedeutung gemäß der allgemeinen Formel I.

Durch die Umsetzung von Mannichbasen der allgemeinen Formel II 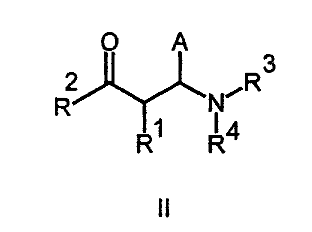 mit substituierten Grignardverbindungen der allgemeinen Formel III, 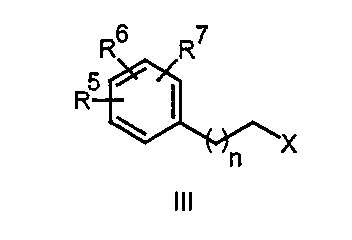 worin n = 1 oder 2 und X = MgCl, MgBr, Mgl oder Li bedeutet, in einem aliphatischen Ether, vorzugsweise Diethylether und/oder Tetrahydrofuran, einem Kohlenwasserstoff, vorzugsweise Hexan oder Toluol, oder Gemischen aus Kohlenwasserstoffen und aliphatischen Ethem, vorzugsweise bei Temperaturen zwischen -70 °C und +110 °C wurden in Abhängigkeit von den Reaktionsbedingungen bevorzugt tertiäre Alkohole mit der relativen Konfiguration der allgemeinen Formel la erhalten,

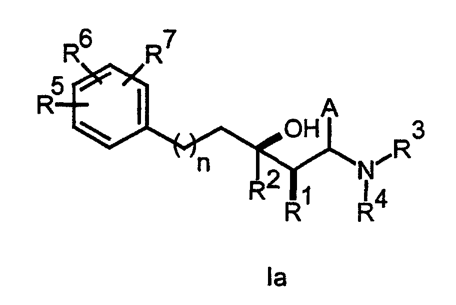

in denen die Aminoarylmethyl- bzw. Aminoheteroarylmethylgruppe *cis* zur Hydroxylgruppe angeordnet ist, wenn R¹ und R² ein Ringsystem bilden. Bei offenkettigen Systemen wird bevorzugt die analoge relative Stereochemie erhalten, die als *anti* zu spezifizieren ist. Die Verbindungen der allgemeinen Formel I lassen sich durch säulenchromatographische Trennung oder durch Kristallisation ihrer Salze, beispielsweise der Hydrochloride, diastereomerenrein erhalten.

Die Mannichbasen der allgemeinen Formel II lassen sich nach literaturbekannten Verfahren (Houben-Weyl - Methoden der Organischen Chemie, E21 b, 1995, S. 1925-1929) durch Umsetzung von Enaminen der allaemeinen Formel IV. 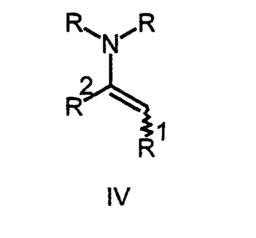 mit einem Imminiumsalz der allgemeinen Formel V, 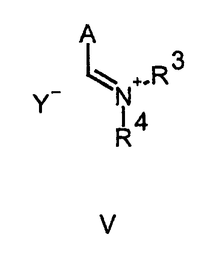 worin Y vorzugsweise Cl⁻, AlCl₄⁻ , Br⁻ oder I⁻ bedeutet, erhalten.

Die Enamine der allgemeinen Formel IV werden nach literaturbekannten Verfahren durch die Umsetzung von Ketonen der allgemeinen Formel VI 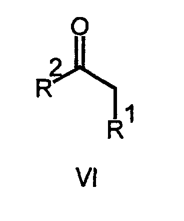 mit sekundären Aminen, vorzugsweise Dimethylamin, Pyrrolidin, Piperidin oder Morpholin erhalten. (Acta Chem. Scand. B38, 1984, S. 49-53). Die Imminiumsalze der allgemeinen Formel V werden nach literaturbekannten Verfahren durch Umsetzung von Aminalen der allgemeinen Formel VII 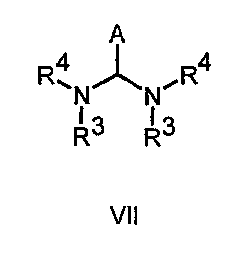 mit Säurechloriden, beispielsweise Acetylchlorid oder Thionylchlorid, hergestellt (Houben-Weyl - Methoden der Organischen Chemie, E21b, 1995, S. 1925-1929).

Die Imminiumsalze der allgemeinen Formel V müssen dabei nicht isoliert werden, sondern können in situ erzeugt und mit Enaminen der allgemeinen Formel IV zu Mannichbasen der allgemeinen Formel II umgesetzt werden (Angew. Chem. 106, 1994, S. 2531-2533). Aufgrund der der Keto-Enol-Tautomerie analogen Enamin-Imin-Tautomerie sind statt der Enamine der allgemeinen Formel IV auch Imine der allgemeinen Formel VIII 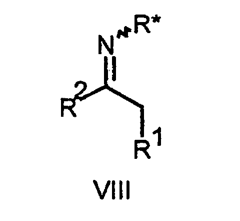 einsetzbar, worin R* für einen Alkyl- oder Aryl-Rest steht. Alternativ dazu können Ketone der allgemeinen Formel VI auch direkt mit Imminiumsalzen der allgemeinen Formel V umgesetzt werden.

Mannichbasen der allgemeinen Formel II können aber auch durch Umsetzung von Enaminen der allgemeinen Formel IV mit einem aromatischen oder heteroaromatischen Aldehyd der allgemeinen Formel IX 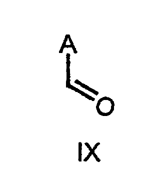 und einem sekundären Amin der allgemeinen Formel HNR³R⁴ (XI), auch in Form des korrespondierenden Hydrochlorids HNR³R⁴·HCl, vorzugsweise in Gegenwart von Triethylamin, Chlortrimethylsilan und Natriumiodid direkt hergestellt werden (Synlett 1997, S. 974-976).

Die Mannichbasen der allgemeinen Formel II werden mit den oben beschriebenen Verfahren in Abhängigkeit von den Reaktionsbedingungen bevorzugt mit der relativen Konfiguration der allgemeinen Formel IIa erhalten, 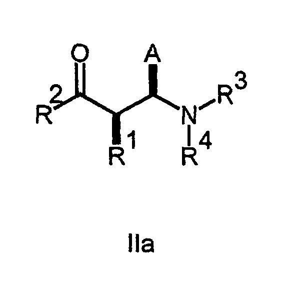 in denen die Aminogruppe *anti* zu R¹ angeordnet ist. Diese Verbindungen der allgemeinen Formel IIa lassen sich durch Kristallisation, auch ihrer Salze, beispielsweise der Hydrochloride, oder durch chromatographische Trennung diastereomerenrein erhalten.

Die Darstellung von Mannichbasen der allgemeinen Formel II durch 1,4-Addition sekundärer Amine der allgemeinen Formel XI an Enone der allgemeinen Formel X, 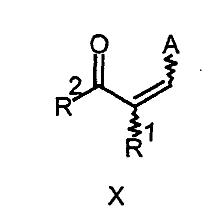 die aus der Aldolkondensation von Ketonen der allgemeinen Formel VI mit aromatischen oder heteroaromatischen Aldehyden der allgemeinen Formel IX erhalten werden, verläuft dagegen weniger stereoselektiv (US-Patent 4,017,637). Diese Vorgehensweise eignet sich daher zur Darstellung der anderen möglichen Stereoisomeren.

Werden chirale Amine zur Darstellung von Enaminen der allgemeinen Formel IV oder Iminen der allgemeinen Formel VIII eingesetzt, so können in der nachfolgenden Mannichreaktion enantiomeren-angereicherte bis enantiomerenreine Mannichbasen der allgemeinen Formel II erhalten werden (Houben-Weyl - Methoden der Organischen Chemie, E21b, 1995, S. 1925-1929).

1-Amino-5-phenylpentan-3-ol- und 1-Amino-6-phenylhexan-3-ol-Verbindungen der allgemeinen Formel I, die ein Phenol enthalten, lassen sich vorzugsweise aus den entsprechenden Methylether-Verbindungen mit Diisobutylaluminiumhydrid in einem aromatischen Kohlenwasserstoff, vorzugsweise Toluol, bei einer Temperatur zwischen 60 und 130 °C herstellen (Synthesis 1975, S. 617-630).

Die Verbindungen der allgemeinen Formel I lassen sich durch Umsetzung mit den entsprechenden Säuren in an sich bekannter Weise in ihre physiologisch verträglichen Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in Methylethylketon.

### Molekularbiologische Untersuchungen:

Die Untersuchungen zur Bestimmung der NMDA antagonistischen Wirkung der jeweiligen Verbindung der allgemeinen Formel I wurde an Hirnmembranhomogenaten (Homogenat von Rattenhim ohne Cerebellum, Pons und Medulla oblongata von männlichen Wistar-Ratten (Charles River, Sulzfeld, Deutschland)) durchgeführt.

Hierzu wurden frisch präparierte Rattengehime nach Abtrennen von Cerebellum, Pons und Medulla oblongata in 50 mmol/l Tris/HCl (pH 7,7) mit einem Polytron-Homogenisator (Modell PT3000, Kinematika AG, Littau, Schweiz) bei 6.000 Umdrehungen pro Minute (UPM) für 1 Minute unter Eiskühlung aufgeschlossen und anschließend für 15 Minuten bei 4 °C und 60.000 g zentrifugiert. Nach Dekantieren und Verwerfen des Überstandes, erneutem Aufnehmen in 50 mmol/l Tris/HCl (pH 7,7) und Aufschluß des Membranpellets mit einem Homogenisator bei 2.000 UPM für 1 Minute wurde erneut für 15 Minuten bei 4 °C und 60.000 g zentrifugiert. Der Überstand wurde wiederum verworfen und das Membranpellet in 50 mmol/l Tris/HCl (pH 7,7) homogenisiert (2.000 UPM für 1 Minute) und aliquotiert bei -70 °C eingefroren.

Für den Rezeptorbindungstest wurden jeweils Aliquote aufgetaut und anschließend für 15 Minuten bei 4 °C und 60.000 g zentrifugiert. Nach Dekantieren und Verwerfen des Überstandes wurde das Membranpellet für den Bindungstest mit Bindungstest-Puffer aufgenommen und homogenisiert (2.000 UPM für 1 Minute). Als Bindungstest-Puffer wurden 5 mmol/l Tris/HCl (pH 7,7) supplementiert mit 30 µmol/l Glycin und 100 µmol/l Glutaminsäure verwendet.

Als radioaktiv markierter Ligand wurde 1 nmol/l (³H)-(+)-MK801 ((5R,10S)-(+)-5-Methyl-10,11-dihydro-5H-dibenzo(a,d)cyclohepten-5,10-imin (NET-972, NEN, Köln Deutschland) zugegeben. Der Anteil an unspezifischer Bindung wurde in Anwesenheit von 10 µmol/l nicht radioaktiv markiertem (+)-MK801 (RBI/Sigma, Deisenhofen, Deutschland) bestimmt. In weiteren Ansätzen wurden die jeweiligen Verbindungen der allgemeinen Formel I in Konzentrationsreihen zugegeben und die Verdrängung des radioaktiven Liganden aus seiner spezifischen Bindung am NMDA-Rezeptor ermittelt. Alle Ansätze wurden als Dreifachbestimmungen ausgeführt. Die Ansätze wurden jeweils für 40 Minuten bei 25 °C im Wasserbad inkubiert und anschließend zur Bestimmung des an das Hirnmembranhomogenat gebundenen radioaktiven Liganden mittels Filtration durch Glasfaserfilter (GF/B) (Typ Whatman GF/B, Hassel, München, Deutschland) geerntet. Die durch die Glasfaserfilterscheiben zurückgehaltene Radioaktivität wurde nach Zugabe von Szintillator (Szintillator "Ready Protein", Beckmann Coulter GmbH, Krefeld, Deutschland) im ß-Counter (Packard TRI-CARB Liquid Szintillation Analyzer 2000CA, Packard Instrument, Meriden, CT 06450, USA) vermessen.

Die aus Dreifachansätzen resultierende prozentuale Hemmung der spezifischen Bindung des Liganden (³H)-(+)-MK801 in Gegenwart von je 10 µmol/l der jeweiligen Verbindung der allgemeinen Formel I dient als Maß für die Affinität dieser Verbindung zu der (+)-MK801-Bindungsstelle des ionotropen NMDA-Rezeptors.

Aus Ansätzen mit Konzentrationsreihen dieser Verbindungen der allgemeinen Formel I wurden IC₅₀-Werte (Konzentration der substituierten Verbindungen mit 50 % Verdrängung des radioaktiven Liganden aus seiner spezifischen Bindung) nach dem Massenwirkungsgesetz mittels nichtlinearer Regression berechnet. Aus diesen IC₅₀-Werten wurden nach der Cheng-Prusoff-Gleichung (Y. Cheng, W.H. Prusoff, 1973, Biochem. Pharmacol., 22, Seiten 3099-3108) Kᵢ-Werte berechnet.

Im folgenden wird die Erfindung anhand von Beispielen erläutert. Diese Beispiele dienen der Erläuterung der Erfindung, schränken aber den allgemeinen Erfindungsgedanken nicht ein.

### Beispiele:

Die Ausbeuten der hergestellten Verbindungen sind nicht optimiert.

Alle Temperaturen sind unkorrigiert.

Als stationäre Phase für die Säulenchromatographie wurde Kieselgel 60 (0.040-0.063 mm) der Firma E. Merck, Darmstadt eingesetzt.

Dünnschicht-chromatographische Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Die Racemattrennungen wurden auf einer Chiracel OD Säule oder auf einer ChiraPak AD Säule mit Vorsäule der Firma Daicel durchgeführt.

Der Begriff Raumtemperatur bedeutet 20 bis 25°C.

Smp. bedeutet Schmelzpunkt (n.b. bedeutet "nicht bestimmt").

SC bedeutet Säulenchromatographie, Hex steht für n-Hexan, EE für Ethylacetat, Ether für Diethylether, Iso für Isopropanol und DEA für Diethylamin.

### Allgemeine Synthesevorschrift 1:

### Grignard-Reaktion

1,2 Moläquivalente Magnesiumspäne wurden in Diethylether oder Tetrahydrofuran p.a. gerührt (ca. 1 ml Lösungsmittel pro mmol Mg). Es wurden 1,2 Moläquivalente des jeweiligen Halogenids, gelöst in 1 ml des Lösungsmittels pro mmol Halogenid, so zugetropft, daß das Reaktionsgemisch leicht siedete. Nach der Zugabe wurde eine Stunde bei Raumtemperatur nachgerührt. Im Falle des unsubstituierten Phenethylgrignards wurde das Grignard-Reagenz alternativ meist nicht hergestellt, sondern eine kommerziell erhältliche Lösung von Phenethylmagnesiumchlorid (1 mol/l in Tetrahydrofuran) (Sigma-Aldrich GmbH, Deisenhofen, Deutschland) vorgelegt. Anschließend wurde 1 Moläquivalent der jeweils eingesetzten Mannichbase in 1,5 ml Lösungsmittel pro mmol gelöst, bei Eisbadkühlung zum Grignard-Ansatz getropft und 15 Stunden bei Raumtemperatur gerührt.

Zur Aufarbeitung wurde gesättigte Ammoniumchloridlösung (1,5 ml pro mmol Mannichbase) bei Eisbadkühlung zugegeben und bei Raumtemperatur dreimal mit Diethylether extrahiert (je 1,5 ml pro mmol Mannichbase). Die vereinigten organischen Extrakte wurden über Natriumsulfat getrocknet, filtriert und am Rotationsverdampfer (500 bis 10 mbar) eingeengt. Zur Reinigung wurde die erhaltene Rohbase in 2-Butanon gelöst (3 ml pro mmol Rohprodukt) und unter Rühren durch Zugabe eines halben Moläquivalents Wasser, gefolgt von 1,1 Moläquivalenten Chlortrimethylsilan das entsprechende Hydrochlorid gefällt.

Bildete sich auch bei Kühlung auf ca. 4 °C und Rühren über Nacht oder nach Zugabe von Ether kein Hydrochlorid, wurde der Fällungsansatz im doppelten Volumen Wasser aufgenommen, mit drei kleinen Portionen Ether gewaschen, die wäßrige Phase mit wenig ca. 30 %iger Natronlauge alkalisch gestellt und dreimal mit Ether extrahiert. Diese letzten Extrakte wurden wiederum vereinigt und entweder direkt einer erneuten Hydrochloridfällung zugeführt oder zuvor durch Säulenchromatographie an Kieselgel gereinigt.

### Allgemeine Synthesevorschrift 2:

### Semiautomatisierte Grignard-Reaktion 1

Wie in der allgemeinen Synthesevorschrift 1 beschrieben wurde aus dem jeweiligen Halogenid und Magnesiumspänen die jeweilige Grignard-Lösung in Tetrahydrofuran hergestellt (theoretischer Gehalt an Grignard-Reagenz 1 mmol/ml). Unter Stickstoffatmosphäre wurden 4,0 ml dieser Lösung in ein mit einer Septumkappe verschlossenes Röhrchen pipettiert. Es wurde auf - 20 °C gekühlt und unter Rühren wurden 2,0 ml der Lösung einer Mannichbase in Tetrahydrofuran (Konzentration 1,60 mmol/ml) zugegeben. Anschließend wurde unter Erwärmung auf Raumtemperatur über Nacht gerührt, bevor erneut auf -20 °C gekühlt und zur Hydrolyse 2,0 ml halbgesättigte Ammoniumchloridlösung zugegeben wurden.

Die weitere Aufarbeitung erfolgte gemäß der allgemeinen Synthesevorschrift 1.
**Beispiel 1:**
   2-(Dimethylaminophenylmethyl)-1-phenethylcyclohexanol, Hydrochlorid
**Beispiel 2:**
   2-[(2-Chlorphenyl)dimethylaminomethyl]-1-phenethylcyclohexanol, Hydrochlorid
**Beispiel 3:**
   2-[(2-Bromphenyl)dimethylaminomethyl]-1-phenethylcyclohexanol, Hydrochlorid
**Beispiel 4:**
   2-[Dimethylamino-(3-methoxyphenyl)methyl]-1-phenethylcyclohexanol, Hydrochlorid
**Beispiel 5:**
   2-(Dimethylamino-o-tolylmethyl)-1-phenethylcyclohexanol, Hydrochlorid
**Beispiel 6:**
   2-(Dimethylaminophenylmethyl)-1-(3-phenylpropyl)cyclohexanol, Hydrochlorid
**Beispiel 7:**
   2-(Dimethylaminophenylmethyl)-1-[2-(2-fluorphenyl)ethyl]cyclohexanol, Hydrochlorid
Beispiel 8:
   2-[Dimethylamino-(2-fluorphenyl)methyl]-1-phenethylcyclohexanol, Hydrochlorid
**Beispiel 9:**
   2-[(2-Chlorphenyl)dimethylaminomethyl]-1-[2-(4-fluorphenyl)ethyl]cyclohexanol, Hydrochlorid
**Beispiel 10:**
   2-[(2-Chlorphenyl)dimethylaminomethyl]-1-[2-(2-fluorphenyl)ethyl]cyclohexanol, Hydrochlorid
**Beispiel 11:**
   2-[(2-Chlorphenyl)dimethylaminomethyl]-1-[2-(3-fluorphenyl)ethyl]cyclohexanol, Hydrochlorid
Beispiel 12:
   1-[2-(2-Chlorphenyl)ethyl]-2-(dimethylaminophenylmethyl)cyclohexanol, Hydrochlorid
**Beispiel 13:**
   1-[2-(3-Chlorphenyl)ethyl]-2-(dimethylaminophenylmethyl)cyclohexanol, Hydrochlorid
**Beispiel 14:**
   1-Phenethyl-2-(phenylpiperidin-1-yl-methyl)cyclohexanol, Hydrochlorid
**Beispiel 15:**
   1-[2-(2-Chlorphenyl)ethyl]-2-(dimethylamino-o-tolylmethyl)cyclohexanol, Hydrochlorid
**Beispiel 16:**
   1-[2-(3-Chlorphenyl)ethyl]-2-(dimethylamino-o-tolylmethyl)cyclohexanol, Hydrochlorid
Beispiel 17:
   2-[Dimethylamino-(2-fluorphenyl)methyl]-1-[2-(4-fluorphenyl)ethyl]cyclohexanol, Hydrochlorid
**Beispiel 18:**
   2-(Dimethylamino-o-tolylmethyl)-1-[2-(4-fluorphenyl)ethyl]cyclohexanol, Hydrochlorid
**Beispiel 19:**
   1-[2-(2-Chlorphenyl)ethyl]-2-[dimethylamino-(2-fluorphenyl)methyl]cyclohexanol, Hydrochlorid
**Beispiel 20:**
   1-[2-(2-Chlorphenyl)ethyl]-2-(dimethylamino-m-tolylmethyl)cyclohexanol, Hydrochlorid
**Beispiel 21:**
   1-[2-(3-Chlorphenyl)ethyl]-2-[dimethylamino-(2-fluorphenyl)methyl]cyclohexanol, Hydrochlorid
**Beispiel 22:**
   1-[2-(2-Chlorphenyl)ethyl]-2-[dimethylamino-(3-fluorphenyl)methyl]cyclohexanol, Hydrochlorid
**Beispiel 23:**
   1-[2-(2-Chlorphenyl)ethyl]-2-(pyrrolidin-1-yl-o-tolylmethyl)cyclohexanol, Hydrochlorid
**Beispiel 24:**
   1-[2-(2-Chlorphenyl)ethyl]-2-(morpholin-4-yl-o-tolylmethyl)cyclohexanol, Hydrochlorid
**Beispiel 25:**
   1-[2-(2-Chlorphenyl)ethyl]-2-(piperidin-1-yl-o-tolylmethyl)cyclohexanol, Hydrochlorid
**Beispiel 26:**
   1-[2-(3-Chlorphenyl)ethyl]-2-(dimethylamino-o-tolylmethyl)cyclohexanol, Hydrochlorid
**Beispiel 27:**
   2-[Dimethylamino-(2-trifluormethylphenyl)methyl]-1-phenethylcyclohexanol, Hydrochlorid
**Beispiel 28:**
   2-[Dimethylamino-(2-methoxyphenyl)methyl]-1-[2-(2-fluorphenyl)ethyl]cyclohexanol, Hydrochlorid
**Beispiel 29:**
   2-[Dimethylamino-(2-fluorphenyl)methyl]-1-[2-(2-fluorphenyl)ethyl]cyclohexanol, Hydrochlorid
**Beispiel 30:**
   2-(Dimethylamino-o-totylmethyl)-1-[2-(2-fluorphenyl)ethyl]cyclohexanol, Hydrochlorid
**Beispiel 31:**
   1-[2-(2-Fluorphenyl)ethyl)-2-(pyrrolidin-1-yl-o-tolylmethyl)cyclohexanol, Hydrochlorid
**Beispiel 32:**
   2-[(2-Bromphenyl)dimethylaminomethyl]-1-[2-(2-chlorphenyl)ethyl]cyclohexanol, Hydrochlorid
**Beispiel 33:**
   2-[(2-Chlorphenyl)dimethylaminomethyl]-1-[2-(2-chlorphenyl)ethyl]cyclohexanol, Hydrochlorid
**Beispiel 34:**
   2-[(2-Bromphenyl)dimethylaminomethyl]-1-[2-(2-fluorphenyl)ethyl]cyclohexanol, Hydrochlorid
**Beispiel 35:**
   2-[(2-Chlorphenyl)dimethylaminomethyl]-1-(2-o-tolylethyl)cyclohexanol, Hydrochlorid
**Beispiel 36:**
   2-[(2-Chlorphenyl)dimethylaminomethyl]-1-(3-phenylpropyl)cyclohexanol, Hydrochlorid
**Beispiel 37:**
   2-(Dimethylaminophenylmethyl)-1-(2-o-tolylethyl)cyclohexanol, Hydrochlorid
**Beispiel 38:**
   2-[(2-Bromphenyl)dimethylaminomethyl]-1-(2-o-tolylethyl)cyclohexanol, Hydrochlorid
**Beispiel 39:**
   2-(Dimethylamino-o-tolylmethyl)-1-(2-o-tolylethyl)cyclohexanol, Hydrochlorid
**Beispiel 40:**
   2-[(2-Bromphenyl)dimethylaminomethyl]-1-(3-phenylpropyl)cyclohexanol, Hydrochlorid
**Beispiel 41:**
   2-[(2-Chlor4-fluorphenyl)dimethylaminomethyl]-1-[2-(2-fluorphenyl)ethyl]cyclohexanol, Hydrochlorid
**Beispiel 42:**
   2-[(2-Chlor4-fluorphenyl)dimethylaminomethyl]-1-[2-(2-chlorphenyl)ethyl]cyclohexanol, Hydrochlorid
**Beispiel 43:**
   2-[Dimethylamino-(2-fluorphenyl)methyl]-1-(2-o-tolylethyl)cyclohexanol, Hydrochlorid
**Beispiel 44:**
   2-[Dimethylamino-(2-fluorphenyl)methyl]-1-(3-phenylpropyl)cyclohexanol, Hydrochlorid
**Beispiel 45:**
   2-(Dimethylamino-o-tolylmethyl)-1-(3-phenylpropyl)cyclohexanol, Hydrochlorid
**Beispiel 46:**
   1-[2-(2-Chlorphenyl)ethyl]-2-[dimethylamino-(2-trifluormethylphenyl)methyl]-cyclohexanol, Hydrochlorid
**Beispiel 47:**
   2-[Dimethylamino-(2-trifluormethylphenyl)methyl]-1-[2-(2-fluorphenyl)ethyl]-cyclohexanol, Hydrochlorid

Die jeweils in den Beispielen 1 bis 47 eingesetzte Mannichbase sowie deren Menge, das jeweils eingesetzte Halogenid sowie die Ausbeuten für die jeweilige, gemäß der allgemeinen Synthesevorschrift 1 oder 2 erhaltenen Beispielverbindung 1 bis 47, deren Aufarbeitung und ggf. deren Schmelzpunkt sind in der nachfolgenden Tabelle 1 wiedergegeben.

**Tabelle 1:**

| Synthese ausgewählter 1-Amino-6-phenyhexan-3-ol bzw. 1-Amino-6-phenylhexan-3-ol-Verbindungen: | | | | | |
|---|---|---|---|---|---|
| **Beispiel** | **Ansatzgröße** | **Mannichbase** | **Halogenid** | **Ausbeute** **(Smp**.) | **Reinigung** |
| **Nr.** | mmol Mannichbase | | | g Hydrochlorid | |
| **1** | 13,0 | 2-(Dimethylaminophenylmethyl)cyclohexanon | Phenethylbromid | 3,26 (100°C)* | Zugabe von Ether |
| **2**** | 7,47 | 2-[(2-Chlorphenyl)dimethylaminomethyl]-cyclohexanon | Phenethylchlorid | 1,12 (110 °C)* | 4°C |
| **3**** | 6,99 | 2-[(2-Bromphenyl)dimethyl- aminomethyl]-cyclohexanon | Phenethylchlorid | 1,13 (162-165 °C) | 4 °C |
| **4**** | 11,5 | 2-[Dimethylamino-(3-methoxyphenyl)-methyl]cyclohexanon | Phenethylchlorid | 3,81 (90 °C)* | Zugabe von Ether |
| **5**** | 10,2 | 2-(Dimethylamino-o-totylmethyl)cyclohexanon | Phenethylchlorid | 2,09 (187-188 °C) | 4 °C |
| **6** | 10,8 | 2-(Dimethylaminophenylmethyl)cyclohexanon | 1-Brom-3-phenylpropan | 2,92 (90 °C)* | Zugabe von Ether |
| **7** | 10,8 | 2-(Dimethylaminophenylmethyl)cyclohexanon | 1-(2-Bromethyl)-2-fluorbenzol fluorbenzol | 2,59 (95 °C)* | Zugabe von Ether |
| **8**** | 8,02 | 2-[Dimethylamino-(2-fluorphenyl)methyl]cyclohexanon | Phenethylchlorid | 1,21 (172-174 °C) | zweite Fällung |
| **9** | 7,52 | 2-[(2-Chlorphenyl)dimethylaminomethyl]cyclohexanon | 1-(2-Bromethyl)-4-fluorbenzol | 1,74 (170°C)* | 4 °C |
| **10** | 22,6 | 2-[(2-Chlorphenyl)dimethylaminomethyl]cyclohexanon | 1-(2-Bromethyl)-2-fluorbenzol | 3,51 (162-163 °C) | SC (Hex/Ether 2/1) |
| **11** | 7,52 | 2-[(2-Chlorphenyl)dimethylaminomethyl]cyclohexanon | 1-(2-Bromethyl)-3-fluorbenzol | 0,24 (120-126 °C) | SC (Hex/EE 4/1) |
| **12** | 86,4 | 2-(Dimethylaminophenylmethyl)cyclohexanon | 1-(2-Bromethyl)-2-chlorbenzol | 3,31 (152°C*) | SC (Hex/Ether 1/1 |
| **13** | 8,64 | 2-(Dimethylaminophenylmethyl)cyclohexanon | 1-(2-Bromethyl)-3-chlorbenzol | 0,84 (95°C)* | zweite Fällung |
| **14**** | 7,37 | 2-(Phenylpiperidin-1-yl-methyl)cyclohexanon | Phenethylchlorid | 1,19 (127 °C)* | zweite Fällung |
| **15** | 40,8 | 2-(Dimethylamino-o-tolylmethyl)cyclohexanon | 1-(2.Bromethyl)-2-chlorbenzol | 3,20 (190-194 °C) | SC (Hex/Ether 1/1) |
| **16***** | 3,19 | 2-(Dimethylamino-o-tolylmethyl)cyclohexanon | 1-(2-Bromethyl)-3chlorbenzo | 0,32 (n.b.) | 4 °C |
| **17***** | 3,19 | 2-[Dimethylamino-(2-fluorphenyl)methyl]cyclohexanon | 1-(2-Bromethyl)-4-fluorbenzol | 0,73 (n.b.) | 4 °C |
| **18***** | 3,19 | 2-(Dimethylamino-o-tolylmethyl)cyclohexanon | 1-(2-Bromethyl)-4-fluorbenzol | 0,57 (n.b.) | 4 °C |
| **19***** | 3,19 | 2-[Dimethylamino-(2-fluorphenyl)methyl]cyclohexanon | 1-(2-Bromethyl)-2-chlorbenzol | 0,50 (213°C) | zweite Fällung |
| **20***** | 3,19 | 2-(Dimethylamino-m-tolylmethyl)cyclohexanon | 1-(2-Bromethyl)-2-chlorbenzol | 0,47 (n.b.) | zweite Fällung |
| **21***** | 3,19 | 2-[Dimethylamino-(2-fluorphenyl)methyl]cyclohexanon | 1-(2-Bromethyl)-3-chlorbenzol | 0,63 (n.b.) | zweite Fällung |
| **22***** | 3,19 | 2-[(Dimethylamino-(3-fluorphenyl)methyl)cyclohexanon | 1-(2-Bromethyl)-2-chlorbenzol | 0,66 (n.b.) | zweite Fällung |
| **23** | 36,8 | 2-(Pyrrolidin-1-yl-o-tolylmethyl)cyclohexanon | 1-(2-Bromethyl)-2-chlorbenzol | 2,18 (155°C)* | SC (Hex/Ether 1/1) |
| **24***** | 3,19 | 2-(Morpholin-4-yl-o-tolylmethyl)cyclohexanon | 1-(2-Bromethyl)-2-chlorbenzol | 0,37 (n.b.) | zweite Fällung |
| **25***** | 3,19 | 2-(Piperidin-1-yl-o-tolylmethyl)cyclohexanon | 1-(2-Bromethyl)-2-chlorbenzol | 0,27 (n.b.) | zweite Fällung |
| **26***** | 3,19 | 2-(Dimethylamino-o-tolylmethyl)cyclohexanon | 1-(2-Bromethyl)-3-chlorbenzol | 0,24 (n.b.) | vierte Fällung |
| **27**** | 8,35 | 2-[Dimethylamino-(2-trifluormethylphenyl)methyl]cyclohexanon | Phenethylchlorid | 2,26 (n.b.) | 4 °C |
| **28** | 9,56 | 2-[Dimethylamino-(2-methoxyphenyl)methyl]cyclohexanon | 1-(2-Bromethyl)-2-fluorbenzol | 0,49 (n.b.) | SC (Ether) |
| **29** | 10,0 | 2-[Dimethylamino-(2-fluorphenyl)methyl]cyclohexanon | 1-(2-Bromethyl)-2-fluorbenzol | 1,53 (n.b.) | SC (Hex/Ether 1/1 ) |
| **30** | 10,2 | 2-(Dimethylamino-o-tolylmethyl)cyclohexanon | 1-(2-Bromethyl)-2-fluorbenzol | 0,52 (n.b.) | SC (Hex/Ether 1/1) |
| **31** | 9,21 | 2-(Pyrrolidin-1-yl-o-tolylmethyl)cyclohexanon | 1-(2-Bromethyl)-2-fluorbenzol | 0,63 (n.b) | SC (Hex/Ether 1/1) |
| **32** | 16,1 | 2-[(2-Bromphenyl)dimethylaminomethyl]cyclohexanon | 1-(2-Bromethyl)-2-chlorbenzol | 3,22 (n.b.) | SC (Hex/Ether 1/1) |
| **33** | 18,8 | 2-[(2-Chlorphenyl)dimethylaminomethyl]cyclohexanon | 1-(2-Bromethyl)-2-chlorbenzol | 3.08 (n.b.) | SC (Hex/Ether 1/1) |
| **34** | 16,1 | 2-[(2-Bromphenyl)dimethylaminomethyl]cyclohexanon | 1-(2-Bromethyl)-2-fluorbenzol | 1,54 (n.b.) | SC (Hex/Ether 1/1) |
| **35** | 18,8 | 2-[(2-Chlorphenyl)dimethylaminomethyl]cyclohexanon | 1-(2-Bromethyl-2-methylbenzol | 1,17 (n.b.) | SC (Hex/Ether 2/1) |
| **36** | 18,8 | 2-[(2-Chlorphenyl)dimethylaminomethyl]cyclohexanon | 1-Brom-3-phenylpropan | 1,11 (n.b.) | SC (Hex/Ether 1/1) |
| **37** | 20,1 | 2-(Dimethylaminophenylmethyl)cyclohexanon | 1-(2-Bromethyl)-2-methyl benzol | 1,30 (n.b.) | SC (Hex/Ether 1/1) |
| **38** | 16,1 | 2-[(2-Bromphenyl)dimethylaminomethyl]cyclohexanon | 1-(2-Bromethyl)-2-methyl benzol | 3,34 (n.b.) | SC (Hex/Ether 2/1) |
| **39** | 20,4 | 2-(Dimethylamino-o-tolylmethyl)cyclohexanon | 1-(2-Bromethyl)-2-methyl benzol | 1,44 (n.b.) | SC (Hex/Ether 1/1) |
| **40** | 16,1 | 2-[(2-Bromphenyl)dimethylaminomethyl]cyclohexanon | 1-Brom-3-phenylpropan | 3,82 (n.b.) | SC (Hex/Ether 2/1) |
| **41** | 17,6 | 2-[(2-Chlor-4-fluorphenyl)dimethylaminomethyl]cyclohexanon | 1-(2-Bromethyl)-2-fluorbenzol | 0,90 (n.b.) | SC (Hex/Ether 2/1) |
| **42** | 17,6 | 2-[(2-Chlor-4-fluorphenyl)dimethylaminomethyl]cyclohexanon | 1-(2-Bromethyl)-2-chlorbenzol | 0,88 (n.b.) | SC (Hex/Ether 2/1) |
| **43** | 20,1 | 2-[Dimethylamino-(2-fluorphenyl)methyl]cyclohexanon | 1-(2-Bromethyl)-2-methyl benzol | 0,79 (n.b.) | SC (Hex/Ether 2/1) |
| **44** | 20,1 | 2-[Dimethylamino-(2-fluorphenyl)methyl]cyclohexanon | 1-Brom-3-phenyl propan | 4,08 (n.b.) | SC (Hex/Ether 2/1) |
| **45** | 20,4 | 2-(Dimethylamino-o-tolylmethyl)cyclohexanon | 1-Brom-3-phenylpropan | 0,63 (n.b.) | SC (EE) |
| **46** | 16,7 | 2-[Dimethylamino-(2-trifluormethylphenyl)methyl]cyclohexanon | 1-(2-Bromethyl)-2-chlorbenzo | 2,08 (n.b.) | SC (Hex/Ether 2/1) |
| **47** | 16,7 | 2-[Dimethylamino-(2-trifluormethylphenyl)methyl]cyclohexanon | 1-(2-Bromethyl)-2-fluorbenzol | 2,06 (n.b.) | SC (Hex/Ether 2/1) |

| | | | | | |
|---|---|---|---|---|---|
| *: Ab dieser Temperatur wurde eine Zersetzung der Verbindung beobachtet. | | | | | |
| **: Verwendet wurde eine kommerziell erhältliche Lösung von Phenethylmagnesiumchlorid in Tetrahydrofuran. | | | | | |
| ***: Die Durchführung erfolgte gemäß der allgemeinen Synthesevorschrift 2. | | | | | |

### Racemattrennungen:

Bei einigen der racemischen Substanzen wurde durch präparative HPLC eine Racemattrennung durchgeführt und anschließend die erhaltenen Fraktionen gemäß der allgemeinen Synthesevorschrift 1 als Hydrochlorid gefällt. Als Elutionsmittel wurde jeweils ein Gemisch aus Hexan, Isopropanol und Diethylamin verwendet. Die Trennbedingungen sind nachfolgend zusammengefaßt:

### Beispiel 15:

| Präparative Trennung: | |
|---|---|
| Elutionsmittel | Hex/Iso/DEA 980:20:1 |
| Säule | ChiraPak AD (10 µm) 250 x 20 mm |
| Probe | 5 Massenprozent in Elutionsmittel/Iso 1:1; 2 ml Probenvolumen je Einspritzung |
| Fluß | 9 ml/min |
| Detektion | 254 nm |

| Analytische Trennung: | |
|---|---|
| Elutionsmittel | Hex/Iso/DEA 980:20:1 |
| Säule | ChiraPak AD (10 µm) 250 x 4,6 mm |
| Probe | 0,1 Massenprozent im Elutionsmittel; 20 µl Probenvolumen |
| Fluß | 1 ml/min |
| Detektion | 235 nm |

### Fraktion 1:

### Beispiel 48:

### (-)-15-1-[2-(2-Chlorphenyl)ethyl]-2-(dimethylamino-o-tolylmethylcyclohexanol, Hydrochlorid

### Fraktion 2:

### Beispiel 49:

### (+)-15-1-[2-(2-Chlorphenyl)ethyl]-2-(dimethylamino-o-tolylmethylcyclohexanol, Hydrochlorid

### Beispiel 6:

| Präparative Trennung: | |
|---|---|
| Elutionsmittel | Hex/Iso/DEA 970:30:1 |
| Säule | Chiracel OD (10 µm) 250 x 20 mm |
| Probe | 5 Massenprozent in Elutionsmittel; 1 ml Probenvolumen je Einspritzung |
| Fluß | 9 ml/min |
| Detektion | 241 nm |

| Analytische Trennung: | |
|---|---|
| Elutionsmittel | Hex/Iso/DEA 970:30:1 |
| Säule | Chiracel OD (10 µm) 250 x 4,6 mm |
| Probe | 0,1 Massenprozent in Elutionsmittel; 20 µl Probenvolumen |
| Fluß | 1 ml/min |
| Detektion | 241 nm |

### Fraktion 1:

### Beispiel 50:

### (-)-2-(Dimethylaminophenylmethyl)-1-(3-phenylpropyl)cyclohexanol, Hydrochlorid

### Fraktion 2:

### Beispiel 51:

### (+)2-(Dimethylaminophenylmethyl)-1-(3-phenylpropyl)cyclohexanol, Hydrochlorid

### Beispiel 10:

| Präparative Trennung: | |
|---|---|
| Elutionsmittel | Hex/Iso/DEA 850:150:1 |
| Säule | Chiracel OD (10 µm) 250 x 25 mm |
| Probe | 5 Massenprozent in Elutionsmittel; 2 ml Probenvolumen je Einspritzung |
| Fluß | 9 ml/min |
| Detektion | 247 nm |

| Analytische Trennung: | |
|---|---|
| Elutionsmittel | Hex/Iso/DEA 850:150:1 |
| Säule | Chiracel OD (10 µm) 250 x 4,6 mm |
| Probe | 0,1 Massenprozent in Elutionsmittel; 20 µl Probenvolumen |
| Fluß | 1 ml/min |
| Detektion | 247 nm |

### Fraktion 1:

### Beispiel 52:

### (- )-2-[(2-Chlorphenyl)dimethylaminomethyl]-1-[2-(2-fluorphenyl)ethyl]cyclohexanol, Hydrochlorid

### Fraktion 2:

### Beispiel 53:

### (+)-2-[(2-Chlorphenyl)dimethylaminomethyl]-1-[2-(2-fluorphenyl)ethyl]cyclohexanol, Hydrochlorid

Der Enantiomerenüberschuß (ee in %) sowie der spezifische Drehwert der erhaltenen Enantiomeren sind in der nachfolgenden Tabelle 2 wiedergegeben.

**Tabelle 2:**

| Enantiomerenüberschuß (ee in %) und spezifische Drehwert der Enantiomeren | | |
|---|---|---|
| **Beispiel** | **Enantiomerenüberschuß** | **Spez. Drehwert** |
| Nr. | ee / % | α_{D}²⁰ in Methanol |
| **48** | ≥ 98 | -34,1 (c =0,935) |
| **49** | ≥ 98 | +33,5 (c = 0,951) |
| **50** | ≥ 98 | -21,2 (c = 0,765) |
| **51** | ≥ 96 | +21,3 (c = 0,712) |
| **52** | ≥ 98 | -34,0 (c = 0,908) |
| **53** | ≥ 98 | +33,8 (c = 0,914) |

### Molekularbiologische Untersuchungen:

Von jeder dieser Beispielverbindungen 1 bis 53 wurde gemäß der obenstehend beschriebenen Vorgehensweise die Affinität zu der (+)-MK801-Bindungsstelle des ionotropen NMDA-Rezeptors bestimmt. Die entsprechende prozentuale Hemmung der spezifischen Bindung des Liganden (³H)-(+)-MK801 bzw. die entsprechenden Kᵢ-Werte sind in der nachfolgenden Tabelle 3 angegeben.

**Tabelle 3:**

| Prozentuale Hemmung der spezifischen Bindung des Liganden (³H)-(+)-MK801 und Kᵢ-Werte. | | |
|---|---|---|
| **Beispiel** | **Prozentuale Hemmung der (**^{**3**}**H)-(+)-MK801-Bindung bei 10 µM** | **K**_{**i**} **/µM** |
| **1** | 56 | 3,8 |
| **2** | 95 | 1,7 |
| **3** | 91 | 1,7 |
| **4** | 51 | 6,2 |
| **5** | 93 | 1,3 |
| **6** | 74 | 2,6 |
| **7** | 91 | 1,1 |
| **8** | 91 | 1,6 |
| **9** | 67 | 3,8 |
| **10** | 102 | 0,4 |
| **11** | 93 | 2,1 |
| **12** | 85 | 1,5 |
| **13** | 61 | 3,1 |
| **14** | 88 | 2,8 |
| **15** | 100 | 0,7 |
| **16** | 92 | 1,9 |
| **17** | 54 | 5,8 |
| **18** | 54 | 6,1 |
| **19** | 102 | 0,8 |
| **20** | 62 | 4,3 |
| **21** | 90 | 2,1 |
| **22** | 74 | 3,0 |
| **23** | 101 | 0,4 |
| **24** | 48 | 3,7 |
| **25** | 89 | 1,4 |
| **26** | 89 | 1,3 |
| **27** | 67 | 4,2 |
| **28** | 82 | 2,5 |
| **29** | 104 | 0,3 |
| **30** | 105 | 0,3 |
| **31** | 99 | 0,1 |
| **32** | 96 | 0,7 |
| **33** | 93 | 0,6 |
| **34** | 95 | 0,4 |
| **35** | 93 | 1,8 |
| **36** | 85 | 1,9 |
| **37** | 77 | 1,8 |
| **38** | 93 | 2,3 |
| **39** | 90 | 1,4 |
| **40** | 82 | 1,9 |
| **41** | 94 | 1,2 |
| **42** | 85 | 2,0 |
| **43** | 92 | 1,4 |
| **44** | 91 | 1,4 |
| **45** | 91 | 1,8 |
| **46** | 79 | 1,9 |
| **47** | 90 | 1,4 |
| **48** | 99 | 0,4 |
| **49** | 93 | 1,3 |
| **50** | 87 | 0,6 |
| **51** | 80 | 2,3 |
| **52** | 105 | 0,2 |
| **53** | 104 | 0,5 |

## Patentansprüche

1. Verwendung wenigstens einer substituierten 1 -Amino-5-phenylpentan-3-olund/oder 1 -Amino-6-phenylhexan-3-ol-Verbindung der allgemeinen Formel I, 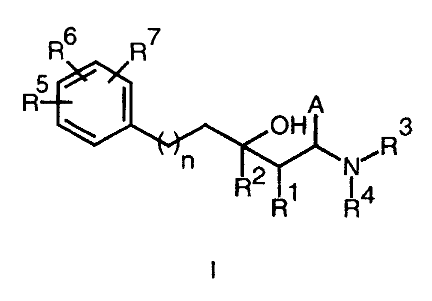 worin jeweils
n = 1 oder 2 ist,
der Rest A einen gegebenenfalls substituierten Aryl- oder Heteroaryl-Rest bedeutet,
die Reste R¹ und R², gleich oder verschieden, für einen C₁₋₆-Alkyl-Rest stehen oder R¹ und R² zusammen eine (CH₂)₂₋₆-Kette bilden, die auch phenylsubstituiert oder benzokondensiert sein kann,
die Reste R³ und R⁴, gleich oder verschieden, für einen C₁₋₆-Alkyl-, einen gegebenenfalls substituierten Aryl-, oder für einen über eine C₁₋₃-Alkylen-Gruppe gebundenen, gegebenenfalls substituierten Aryl-Rest stehen oder R³ und R⁴ zusammen (CH₂)₃₋₆ oder CH₂CH₂OCH₂CH₂ bedeuten,
die Reste R⁵, R⁶, R⁷, gleich oder verschieden, H, F, Cl, Br, I, CF₃, OR⁸, SO₂CH₃, SO₂CF₃, Phenyl, CN, NO₂ oder einen C₁₋₆-Alkylrest bedeuten,
der Rest R⁸ für H, einen C₁₋₆-Alkyl-, einen gegebenenfalls substituierten Aryl-, einen gegebenfalls substituierten Heteroaryl- oder für einen über eine C₁₋₃-Alkylen-Gruppe gebundenen, gegebenenfalls substituierten Aryl- oder Heteroaryl-Rest steht,
und/oder wenigstens eines ihrer Diastereomeren und/oder eines ihrer Enantiomeren und/oder eines der entsprechenden physiologisch verträglichen Salze zur Herstellung eines Arzneimittels mit NMDA antagonistischer Wirkung zur Prophylaxe von Schlaganfällen oder zur Behandlung einer Krankheit ausgewählt aus der Gruppe bestehend aus Epilepsie, Schizophrenie, neurodegenerativen Erkrankungen, insbesondere Morbus Alzheimer, Morbus Huntington oder Morbus Parkinson, cerebraler Ischämie, cerebralem Infarkt, Psychosen bedingt durch erhöhten Aminosäurespiegel, Hirnödem, Unterversorgungszuständen des zentralen Nervensystems, insbesondere Hypoxie oder Anoxie, AIDS-Demens, Encephalomyelitis, Tourette-Syndrom, perinataler Asphyxie und Tinnitus.

2. Verwendung nach Anspruch 1 **dadurch gekennzeichnet, daß** die Reste R¹ und R², gleich oder verschieden, für einen C₁₋₃-Alkyl-Rest stehen und die Reste R³ bis R⁸ und A die Bedeutung gemäß Anspruch 1 haben.

3. Verwendung nach Anspruch 1 **dadurch gekennzeichnet, daß** die Reste R³ und R⁴, gleich oder verschieden, für einen C₁₋₃-Alkyl-Rest stehen und die Reste R¹, R², R⁵ bis R⁸ und A die Bedeutung gemäß Anspruch 1 haben.

4. Verwendung nach Anspruch 1 **dadurch gekennzeichnet, daß** der Rest R⁸ für einen C₁₋₃-Alkyl-Rest steht und die Reste R¹ bis R⁷ und A die Bedeutung gemäß Anspruch 1 haben.

5. Verwendung nach Anspruch 1 **dadurch gekennzeichnet, daß** die Reste R¹ und R² zusammen eine (CH₂)₂₋₆-Kette bilden, die auch phenylsubstituiert oder benzokondensiert sein kann, und die Reste R³ bis R⁸ und A die Bedeutung gemäß Anspruch 1 haben.

6. Verwendung nach Anspruch 1, **dadurch gekennzeichnet, daß** A einen unsubstituierten oder substituierten Phenyl-, Thiophenyl- oder Furyl-Rest bedeutet und die Reste R¹ bis R⁸ die Bedeutung gemäß Anspruch 1 haben.

7. Verwendung nach Anspruch 1, **dadurch gekennzeichnet, daß** die Reste R⁵ bis R⁷, gleich oder verschieden, H einen Halogen- oder einen CF₃-Rest bedeuten und die Reste R¹ bis R⁴, R⁸ und A die Bedeutung gemäß Anspruch 1 haben.

8. Verwendung nach Anspruch 1, **dadurch gekennzeichnet, daß** die Reste R¹ und R² zusammen einen Cyclohexylring bilden, der auch phenylsubstituiert oder benzokondensiert sein kann, A einen substituierten oder unsubstituierten Phenyl-, Thiophenyl oder Furyl bedeutet und die Reste R³ bis R⁸ die Bedeutung gemäß Anspruch 1 haben.

9. Verwendung nach Anspruch 1, **dadurch gekennzeichnet, daß** der Phenylring der allgemeinen Formel I ein- oder zweifach in ortho-Position substituiert ist und die Reste R¹ bis R⁸ und A die Bedeutung gemäß Anspruch 1 haben.

10. Verwendung nach Anspruch 1, **dadurch gekennzeichnet, daß** A einen Phenylring darstellt, der ein- oder zweifach in ortho-Position substituiert ist, und die Reste R¹ bis R⁸ die Bedeutung gemäß Anspruch 1 haben.

11. Verwendung nach Anspruch 1, **dadurch gekennzeichnet, daß** A einen Phenylring darstellt, der ein- oder zweifach in ortho-Position substituiert ist, und der Phenylring der allgemeinen Formel I ein- oder zweifach in ortho-Position substituiert ist, und die Reste R¹ bis R⁸ die Bedeutung gemäß Anspruch 1 haben.

12. Verwendung nach Anspruch 1, **dadurch gekennzeichnet, daß** als Verbindung der allgemeinen Formel I wenigstens eine der nachfolgenden Verbindungen vorliegt:
2-(Dimethylaminophenylmethyl)-1-phenethylcyclohexanol oder das entsprechende Hydrochlorid,
2-[(2-Chlorphenyl)dimethylaminomethyl]-1-phenethylcyclohexanol oder das entsprechende Hydrochlorid,
2-[(2-Bromphenyl)dimethylaminomethyl]-1-phenethylcyclohexanol oder das entsprechende Hydrochlorid,
2-[Dimethylamino-(3-methoxyphenyl)methyl]-1-phenethylcyclohexanol oder das entsprechende Hydrochlorid,
2-(Dimethylamino-o-tolylmethyl)-1-phenethylcyclohexanol oder das entsprechende Hydrochlorid,
2-(Dimethylaminophenylmethyl)-1-(3-phenylpropyl)cyclohexanol oder das entsprechende Hydrochlorid,
2-(Dimethylaminophenylmethyl)-1-[2-(2-fluorphenyl)ethyl]cyclohexanol oder das entsprechende Hydrochlorid,
2-[Dimethylamino-(2-fluorphenyl)methyl]-1-phenethylcyclohexanol oder das entsprechende Hydrochlorid,
2-[(2-Chlorphenyl)dimethylaminomethyl)-1-[2-(4-fluorphenyl)ethyl]cyclohexanol oder das entsprechende Hydrochlorid,
2-[(2-Chlorphenyl)dimethylaminomethyl]-1-[2-(2-fluorphenyl)ethyl]cyclohexanol oder das entsprechende Hydrochlorid,
2-[(2-Chlorphenyl)dimethylaminomethyl]-1-[2-(3-fluorphenyl)ethyl]cyclohexanol oder das entsprechende Hydrochlorid,
1-[2-(2-Chlorphenyl)ethyl]-2-(dimethylaminophenylmethyl)cyclohexanol oder das entsprechende Hydrochlorid,
1-[2-(3-Chlorphenyl)ethyl]-2-(dimethylaminophenylmethyl)cyclohexanol oder das entsprechende Hydrochlorid,
1-Phenethyl-2-(phenylpiperidin-1-yl-methyl)cyclohexanol oder das entsprechende Hydrochlorid,
1-[2-(2-Chlorphenyl)ethyl]-2-(dimethylamino-o-tolylmethyl)cyclohexanol oder das entsprechende Hydrochlorid,
1-[2-(3-Chlorphenyl)ethyl]-2-(dimethylamino-o-tolylmethyl)cyclohexanol oder das entsprechende Hydrochlorid,
2-[Dimethylamino-(2-fluorphenyl)methyl]-1-[2-(4-fluorphenyl)ethyl]cyclohexanol oder das entsprechende Hydrochlorid,
2-(Dimethylamino-o-tolylmethyl)-1-[2-(4-fluorphenyl)ethyl]cyclohexanol oder das entsprechende Hydrochlorid,
1-[2-(2-Chlorphenyl)ethyl]-2-[dimethylamino-(2-fluorphenyl)methyl]cyclohexanol oder das entsprechende Hydrochlorid,
1-[2-(2-Chlorphenyl)ethyl]-2-(dimethylamino-m-tolylmethyl)cyclohexanol oder das entsprechende Hydrochlorid,
1-[2-(3-Chlorphenyl)ethyl]-2-[dimethylamino-(2-fluorphenyl)methyl]cyclohexanol oder das entsprechende Hydrochlorid,
1-[2-(2-Chlorphenyl)ethyl]-2-[dimethylamino-(9-fluorphenyl)methyl]cyclohexanol oder das entsprechende Hydrochlorid,
1-[2-(2-Chlorphenyl)ethyl]-2-(pyrrolidin-1-yl-o-tolylmethyl)cyclohexanol oder das entsprechende Hydrochlorid,
1-[2-(2-Chlorphenyl)ethyl]-2-(morpholin-4-yl-o-tolylmethyl)cyclohexanol oder das entsprechende Hydrochlorid,
1-[2-(2-Chlorphenyl)ethyl]-2-(piperidin-1-yl-o-tolylmethyl)cyclohexanol oder das entsprechende Hydrochlorid,
1-[2-(3-Chlorphenyl)ethyl]-2-(dimethylamino-o-tolylmethyl)cyclohexanol oder das entsprechende Hydrochlorid,
2-[Dimethylamino-(2-trifluormethylphenyl)methyl]-1-phenethylcyclohexanol oder das entsprechende Hydrochlorid,
2-[Dimethylamino-(2-methoxyphenyl)methyl]-1-[2-(2-fluorphenyl)ethyl]cyclohexanol oder das entsprechende Hydrochlorid,
2-(Dimethylamino-(2-fluorphenyl)methyl]-1-[2-(2-fluorphenyl)ethylcyclohexanol oder das entsprechende Hydrochlorid,
2-(Dimethylamino-o-tolylmethyl)-1-[2-(2-fluorphenyl)ethyl]cyclohexanol oder das entsprechende Hydrochlorid,
1-[2-(2-Fluorphenyl)ethyl]-2-(pyrrolidin-1-yl-o-tolylmethyl)cyclohexanol oder das entsprechende Hydrochlorid,
2-[(2-Bromphenyl)dimethylaminomethyl]-1-[2-(2-chlorphenyl)ethyl)cyclohexanol oder das entsprechende Hydrochlorid,
2-[(2-Chlorphenyl)dimethylaminomethyl]-1-[2-(2-chlorphenyl)ethyl]cyclohexanol oder das entsprechende Hydrochlorid,
2-[(2-Bromphenyl)dimethylaminomethyl]-1-[2-(2-fluorphenyl)ethyl]cyclohexanol oder das entsprechende Hydrochlorid,
2-[(2-Chlorphenyl)dimethylaminomethyl]-1-(2-o-tolylethyl)cyclohexanol oder das entsprechende Hydrochlorid,
2-[(2-Chlorphenyl)dimethylaminomethyl]-1-(3-phenylpropyl)cyclohexanol oder das entsprechende Hydrochlorid,
2-(Dimethylaminophenylmethyl)-1-(2-o-tolylethyl)cyclohexanol oder das entsprechende Hydrochlorid,
2-[(2-Bromphenyl)dimethylaminomethyl]-1-(2-o-tolylethyl)cyclohexanol oder das entsprechende Hydrochlorid,
2-(Dimethylamino-o-tolylmethyl)-1-(2-o-tolylethyl)cyclohexanol oder das entsprechende Hydrochlorid,
2-[(2-Bromphenyl)dimethylaminomethyl]-1-(3-phenylpropyl)cyclohexanol oder das entsprechende Hydrochlorid,
2-[(2-Chlor4-fluorphenyl)dimethylaminomethyl]-1-[2-(2-fluorphenyl)ethyl]cyclohexanol oder das entsprechende Hydrochlorid,
2-[(2-Chlor4-fluorphenyl)dimethylaminomethyl]-1-[2-(2-chlorphenyl)ethyl]cyclohexanol oder das entsprechende Hydrochlorid,
2-[Dimethylamino-(2-fluorphenyl)methyl]-1-(2-o-tolylethyl)cyclohexanol oder das entsprechende Hydrochlorid,
2-[Dimethylamino-(2-fluorphenyl)methyl]-1-(3-phenylpropyl)cyclohexanol oder das entsprechende Hydrochlorid,
2-(Dimethylamino-o-tolylmethyl)-1-(3-phenylpropyl)cyclohexanol oder das entsprechende Hydrochlorid,
1-[2-(2-Chlorphenyl)ethyl]-2-[dimethylamino-(2-trifluormethylphenyl)methyl]-cyclohexanol oder das entsprechende Hydrochlorid,
oder
2-[Dimethylamino-(2-trifluormethylphenyl)methyl]-1-[2-(2-fluorphenyl)ethyl]-cyclohexanol oder das entsprechende Hydrochlorid.

13. Verwendung gemäß einem der Ansprüche 1 bis 12, **dadurch gekennzeichnet, daß** man ein Gemisch der Enantiomeren wenigstens einer Verbindung gemäß der allgemeinen Formel I einsetzt, wobei die Enantiomeren nicht in äquimolaren Mengen vorliegen.

14. Verwendung gemäß Anspruch 13, **dadurch gekennzeichnet, daß** eines der Enantiomere einen relativen Anteil von 5 bis 45 Massenprozent an dem Enantiomerengemisch hat.

## Claims

1. Use of at least one substituted 1-amino-5-phenylpentane-3-ol and/or 1-amino-6-phenylhexane-3-ol compound with general formula I, 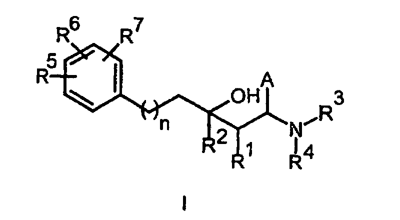 where
n = 1 or 2,
group A represents an optionally substituted aryl or heteroaryl group,
groups R¹ and R², the same or different, stand for a C₁₋₆ alkyl group or groups R¹ and R² together form a (CH₂)₂₋₆ chain, which may also be phenyl substituted or benzo-condensed,
groups R³ and R⁴, the same or different, stand for a C₁₋₆ alkyl group, an optionally substituted aryl group or for a C₁₋₃ alkyl group-bound, optionally substituted aryl group or groups R³ and R⁴ together represent (CH₂)₃₋₆ or CH₂CH₂OCH₂CH₂,
groups R⁵, R⁶ and R⁷, the same or different, represent H, F, Cl, Br, I, CF₃, OR⁸, SO₂CH₃, SO₂CF₃, phenyl, CN, NO₂ or a C₁₋₆ alkyl group, preferably a C₁₋₃ alkyl group,
group R⁸ stands for H, a C₁₋₆ alkyl group, an optionally substituted aryl group, an optionally substituted heteroaryl group or for a C₁₋₃ alkyl group-bound, optionally substituted aryl or heteroaryl group,
and/or at least one of the diastereomers thereof and/or one of the enantiomers thereof and/or a corresponding physiologically tolerable salt for the manufacture of a medicament with an NMDA antagonistic effect for the prevention of cerebrovascular attacks (strokes) or for the treatment of a disease selected from the group comprising: epilepsy, schizophrenia, neurodegenerative diseases, in particular Alzheimer's disease, Huntington's disease or Parkinson's disease, cerebral ischaemia, cerebral infarction, psychoses caused by raised amino acid level, cerebral oedema, under-supply of the central nervous system, in particular hypoxia or anoxia, AIDS dementia, encephalomyelitis, Tourette's syndrome, perinatal asphyxia and tinnitus.

2. Use according to Claim 1 **characterised in that** groups R¹ and R², the same or different, stand for a C₁₋₃-alkyl group and groups R³ to R⁸ and A have the meaning in accordance with Claim 1.

3. Use according to Claim 1 **characterised in that** groups R³ and R⁴, the same or different, stand for a C₁₋₃-alkyl group and groups R¹, R², R⁵ to R⁸ and A have the meaning in accordance with Claim 1.

4. Use according to Claim 1 **characterised in that** group R⁸ stands for a C₁₋₃-alkyl group and groups R¹ to R⁷ and A have the meaning in accordance with Claim 1.

5. Use according to Claim 1 **characterised in that** groups R¹ and R² together form a (CH₂)₂₋₆ chain, which may also be phenyl substituted or benzo-condensed, and groups R³ to R⁸ and A have the meaning in accordance with Claim 1.

6. Use according to Claim 1 **characterised in that** A means an unsubstituted or substituted phenyl, thiophenyl or furyl group and groups R¹ to R⁸ have the meaning in accordance with Claim 1.

7. Use according to Claim 1 **characterised in that** groups R⁵ to R⁷, the same or different, mean H, a halogen or a CF₃ group and groups R¹ to R⁴, R⁸ and A have the meaning in accordance with Claim 1.

8. Use according to Claim 1 **characterised in that** groups R¹ and R² together form a cyclohexyl ring, which may also be phenyl substituted or benzo-condensed, A means a substituted or unsubstituted phenyl, thiophenyl or furyl and groups R³ to R⁸ have the meaning in accordance with Claim 1.

9. Use according to Claim 1 **characterised in that** the phenyl ring of general formula I is substituted once or twice in an ortho-position and groups R¹ to R⁸ have the meaning in accordance with Claim 1.

10. Use according to Claim 1 **characterised in that** A represents a phenyl ring, which is substituted once or twice in an ortho-position, and groups R¹ to R⁸ have the meaning in accordance with Claim 1.

11. Use according to Claim 1 **characterised in that** A represents a phenyl ring, which is substituted once or twice in an ortho-position, the phenyl ring of general formula I is substituted once or twice in an ortho-position, and groups R¹ to R⁸ have the meaning in accordance with Claim 1.

12. Use according to Claim 1 **characterised in that** at least one of the following compounds is present as the compound of general formula I:
2-(dimethylaminophenylmethyl)-1-phenethylcyclohexanol or the corresponding hydrochloride,
2-[(2-chlorophenyl)dimethylaminomethyl]-1-phenethylcyclohexanol or the corresponding hydrochloride,
2-[(2-bromophenyl)dimethylaminomethyl)-1-phenethylcyclohexanol or the corresponding hydrochloride,
2-[dimethylamino-(3-methoxyphenyl)methyl]-1-phenethylcyclohexanol or the corresponding hydrochloride,
2-(dimethylamino-o-tolylmethyl)-1-phenethylcyclohexanol or the corresponding hydrochloride
2-(dimethylaminophenylmethyl)-1-(3-phenylpropyl)cyclohexanol or the corresponding hydrochloride,
2-(dimethylaminophenylmethyl)-1-[2-(2-fluorophenyl)ethyl]cyclohexanol or the corresponding hydrochloride,
2-[dimethylamino-(2-fluorophenyl)methyl]-1-phenethylcyclohexanol or the corresponding hydrochloride,
2-[(2-chlorophenyl)dimethylaminomethyl]-1-[2-(4-fluorophenyl)ethyl]cyclohexanol or the corresponding hydrochloride,
2-[(2-chlorophenyl)dimethylaminomethyl]-1-[2-(2-fluorophenyl)ethyl]cyclohexanol or the corresponding hydrochloride,
2-[(2-chlorophenyl)dimethylaminomethyl]-1-[2-(3-fluorophenyl)ethyl]cyclohexanol or the corresponding hydrochloride,
1-[2-(2-chlorophenyl)ethyl]-2-(dimethylaminophenylmethyl)cyclohexanol or the corresponding hydrochloride,
1-[2-(3-chlorophenyl)ethyl]-2-(dimethylaminophenylmethyl)cyclohexanol or the corresponding hydrochloride,
1-phenethyl-2-(phenylpiperidine-1-yl-methyl)cyclohexanol or the corresponding hydrochloride,
1-[2-(2-chlorophenyl)ethyl]-2-(dimethylamino-o-tolylmethyl)cyclohexanol or the corresponding hydrochloride,
1-[2-(3-chlorophenyl)ethyl]-2-(dimethylamino-o-tolylmethyl)cyclohexanol or the corresponding hydrochloride,
2-[dimethylamino-(2-fluorophenyl)methyl]-1-[2-(4-fluorophenyl)ethyl]cyclohexanol or the corresponding hydrochloride,
2-(dimethylamino-o-tolylmethyl)-1-[2-(4-fluorophenyl)ethyl]cyclohexanol or the corresponding hydrochloride,
1-[2-(2-chlorophenyl)ethyl]-2-(dimethylamino-(2-fluorophenyl)methyl]cyclohexanol or the corresponding hydrochloride,
1-[2-(2-chlorophenyl)ethyl]-2-(dimethylamino-m-tolylmethyl)cyclohexanol or the corresponding hydrochloride,
1-[2-(3-chlorophenyl)ethyl]-2-(dimethylamino-(2-fluorophenyl)methyl]cyclohexanol or the corresponding hydrochloride,
1-[2-(2-chlorophenyl)ethyl]-2-(dimethylamino-(3-fluorophenyl)methyl]cyclohexanol or the corresponding hydrochloride,
1-[2-(2-chlorophenyl)ethyl]-2-(pyrrolidine-1-yl-o-tolylmethyl)cyclohexanol or the corresponding hydrochloride,
1-[2-(2-chlorophenyl)ethyl]-2-(morpholine-4-yl-o-tolylmethyl)cyclohexanol or the corresponding hydrochloride,
1-[2-(2-chlorophenyl)ethyl]-2-(piperidine-1-yl-o-tolylmethyl)cyclohexanol or the corresponding hydrochloride,
1-[2-(3-chlorophenyl)ethyl]-2-(dimethylamino-o-tolylmethyl)cyclohexanol or the corresponding hydrochloride,
2-[dimethylamino-(2-trifluoromethylphenyl)methyl]-1-phenethylcyclohexanol or the corresponding hydrochloride,
2-[dimethylamino-(2-methoxyphenyl)methyl]-1-[2-(2-fluorophenyl)ethyl]cyclohexanol or the corresponding hydrochloride,
2-[dimethylamino-(2-fluorophenyl)methyl]-1-[2-(2-fluorophenyl)ethyl]cyclohexanol or the corresponding hydrochloride,
2-(dimethylamino-o-tolylmethyl)-1-[2-(2-fluorophenyl)ethyl]cyclohexanol or the corresponding hydrochloride,
1-[2-(2-fluorophenyl)ethyl]-2-(pyrrolidine-1-yl-o-tolylmethyl)cyclohexanol or the corresponding hydrochloride,
2-[(2-bromophenyl)dimethylaminomethyl]-1-[2-(2-chlorophenyl)ethyl]cyclohexanol or the corresponding hydrochloride,
2-[(2-chlorophenyl)dimethylaminomethyl]-1-[2-(2-chlorophenyl)ethyl)cyclohexanol or the corresponding hydrochloride,
2-[(2-bromophenyl)dimethylaminomethyl]-1-[2-(2-fluorophenyl)ethyl]cyclohexanol or the corresponding hydrochloride,
2-[(2-chlorophenyl)dimethylaminomethyl]-1-(2-o-tolylethyl)cyclohexanol or the corresponding hydrochloride,
2-[(2-chlorophenyl)dimethylaminomethyl]-1-(3-phenylpropyl)cyclohexanol or the corresponding hydrochloride,
2-(dimethylaminophenylmethyl)-1-(2-o-tolylethyl)cyclohexanol or the corresponding hydrochloride,
2-[(2-bromophenyl)dimethylaminomethyl]-1-(2-o-tolylethyl)cyclohexanol or the corresponding hydrochloride,
2-(dimethylamino-o-tolylmethyl]-1-(2-o-tolylethyl)cyclohexanol or the corresponding hydrochloride,
2-[(2-bromophenyl)dimethylaminomethyl]-1-(3-phenylpropyl)cyclohexanol or the corresponding hydrochloride,
2-[(2-chloro4-fluorophenyl)dimethylaminomethyl]-1-[2-(2-fluorophenyl)ethyl]cyclohexanol or the corresponding hydrochloride,
2-[(2-chloro4-fluorophenyl)dimethylaminomethyl]-1-[2-(2-chlorophenyl)ethyl]cyclohexanol or the corresponding hydrochloride,
2-[dimethylamino-(2-fluorophonyl)methyl]-1-(2-o-tolylethyl)cyclohexanol or the corresponding hydrochloride,
2-[dimethylamino-(2-fluorophenyl)methyl]-1-(3-phenylpropyl)cyclohexanol or the corresponding hydrochloride,
2-(dimethylamino-o-tolylmethyl)-1-(3-phenylpropyl)cyclohexanol or the corresponding hydrochloride,
1-[2-(2-chlorophenyl)ethyl]-2-[dimethylamino-(2-trifluoromethylphenyl)methyl]cyclohexanol or the corresponding hydrochloride,
or
2-[dimethylamino-(2-trifluoromethylphenyl)methyl]-1-[2-(2-fluorophenyl)ethyl]cyclohexanol or the corresponding hydrochloride.

13. Use according to one of Claims 1 to 30 **characterised in that** a mixture of enantiomers of at least one compound according to general formula I is used, whereby the enantiomers are not present in equimolar quantities.

14. Use according to Claim 31 **characterised in that** one of the enantiomers has a relative proportion of 5 to 45 percent by mass of the enantiomer mixture.

## Revendications

1. Utilisation d'au moins un dérivé de 1-amino-5-phénylpentan-3-ol et/ou de 1-amino-6-phénylhexan-3-ol substitué de formule générale I 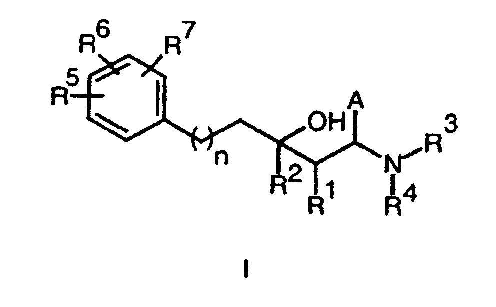 dans laquelle chaque fois
n = 1 ou 2,
le reste A représente un radical aryle ou hétéroaryle éventuellement substitué,
les restes R¹ et R², identiques ou différents, représentent un radical alkyle en C₁₋₆, ou R¹ et R² forment ensemble une chaîne (CH₂)₂₋₆ qui peut également être substituée par un groupe phényle ou condensée avec un noyau benzénique,
les restes R³ et R⁴, identiques ou différents, représentent un radical alkyle en C₁₋₆, un radical aryle éventuellement substitué ou un radical aryle éventuellement substitué, relié par un groupe alkylène en C₁₋₃, ou R³ et R⁴ représentent ensemble un groupe (CH₂)₃₋₆ ou CH₂CH₂OCH₂CH₂,
les restes R⁵, R⁶, R⁷, identiques ou différents, représentent H, F, Cl, Br, I, CF₃, OR⁸, SO₂CH₃, SO₂CF₃, le groupe phényle, CN, NO₂ ou un radical alkyle en C₁₋₆,
le reste R⁸ représente H, un radical alkyle en C₁₋₆, un radical aryle éventuellement substitué, un radical hétéroaryle éventuellement substitué, ou un radical aryle ou hétéroaryle éventuellement substitué, relié par un groupe alkylène en C₁₋₃,
et/ou d'au moins un de ses diastéréoisomères et/ou un de ses énantiomères et/ou d'un des sels correspondants, physiologiquement acceptables, pour la fabrication d'un médicament à action antagoniste du NMDA (N-méthyl-D-aspartate), destiné à la prophylaxie d'accidents vasculaires cérébraux ou au traitement d'une maladie choisie dans le groupe constitué par l'épilepsie, la schizophrénie, les maladies neurodégénératives, en particulier la maladie d'Alzheimer, la maladie de Huntington ou le maladie de Parkinson, l'ischémie cérébrale, l'infarctus cérébral, les psychoses dues à des taux élevés d'aminoacides, l'oedème cérébral, des états d'alimentation insuffisante du système nerveux central, en particulier l'hypoxie ou l'anoxie, la démence due au SIDA, la céphalomyélite, le syndrome de Tourette, l'asphyxie périnatale et l'acouphène.

2. Utilisation selon la revendication 1, **caractérisée en ce que** les restes R¹ et R², identiques ou différents, représentent un radical alkyle en C₁₋₃, et les restes R³ à R⁸ et A ont les significations selon la revendication 1.

3. Utilisation selon la revendication 1, **caractérisée en ce que** les restes R³ et R⁴, identiques ou différents, représentent un radical alkyle en C₁₋₃, et les restes R¹, R², R⁵ à R⁸ et A ont les significations selon la revendication 1.

4. Utilisation selon la revendication 1, **caractérisée en ce que** le reste R⁸ représente un radical alkyle en C₁₋₃ et les restes R¹ à R⁷ et A ont les significations selon la revendication 1.

5. Utilisation selon la revendication 1, **caractérisée en ce que** les restes R¹ et R² forment ensemble une chaîne (CH₂)₂₋₆ qui peut également être substituée par un groupe phényle ou condensée à un noyau benzénique, et les restes R³ à R⁸ et A ont les significations selon la revendication 1.

6. Utilisation selon la revendication 1, **caractérisée en ce que** A représente un radical phényle, thiophényle ou furyle, substitué ou non substitué, et les restes R¹ à R⁸ ont les significations selon la revendication 1.

7. Utilisation selon la revendication 1, **caractérisée en ce que** les restes R⁵ à R⁷, identiques ou différents, représentent un atome d'hydrogène ou d'halogène ou un radical CF₃, et les restes R¹ à R⁴, R⁸ et A ont les significations selon la revendication 1.

8. Utilisation selon la revendication 1, **caractérisée en ce que** les restes R¹ et R² forment ensemble un cycle cyclohexyle qui peut également être substitué par un groupe phényle ou condensé à un noyau benzénique, A représente un radical phényle, thiophényle ou furyle, substitué ou non substitué, et les restes R³ à R⁸ ont les significations selon la revendication 1.

9. Utilisation selon la revendication 1, **caractérisée en ce que** le cycle phényle de la formule générale I est une ou deux fois substitué en position ortho et les restes R¹ à R⁸ et A ont les significations selon la revendication 1.

10. Utilisation selon la revendication 1, **caractérisé en ce que** A représente un cycle phényle qui est une ou deux fois substitué en position ortho, et les restes R¹ à R⁸ ont les significations selon la revendication 1.

11. Utilisation selon la revendication 1, **caractérisée en ce que** A représente un cycle phényle qui est une ou deux fois substitué en position ortho, et le cycle phényle de la formule générale I est une ou deux fois substitué en position ortho, et les restes R¹ à R⁸ ont les significations selon la revendication 1.

12. Utilisation selon la revendication 1, **caractérisée en ce que**, en tant que dérivé de formule générale I, est présent au moins l'un des composés suivants :
2-(diméthylaminophénylméthyl)-1-phénéthylcyclohexanol ou le chlorhydrate correspondant,
2-[(2-chlorophényl)diméthylaminométhyl]-1-phénéthylcyclohexanol ou le chlorhydrate correspondant,
2-[(2-bromophényl)diméthylaminométhyl]-1-phénéthylcyclohexanol ou le chlorhydrate correspondant,
2-[diméthylamino-(3-méthoxyphényl)méthyl]-1-phénéthylcyclohexanol ou le chlorhydrate correspondant,
2-(diméthylamino-o-tolylméthyl)-1-phénéthylcyclohexanol ou le chlorhydrate correspondant,
2-(diméthylaminophénylméthyl)-1-(3-phénylpropyl)-cyclohexanol ou le chlorhydrate correspondant,
2-(diméthylaminophénylméthyl)-1-[2-(2-fluorophényl)éthyl]cyclohexanol ou le chlorhydrate correspondant,
2-[diméthylamino-(2-fluorophényl)méthyl]-1-phénéthylcyclohexanol ou le chlorhydrate correspondant,
2-[(2-chlorophényl)diméthylaminométhyl]-1-[2-(4-fluorophényl)éthyl]cyclohexanol ou le chlorhydrate correspondant,
2-[(2-chlorophényl)diméthylaminométhyl]-1-[2-(2-fluorophényl)éthyl]cyclohexanol ou le chlorhydrate correspondant,
2-[(2-chlorophényl)diméthylaminométhyl]-1-[2-(3-fluorophényl)éthyl]cyclohexanol ou le chlorhydrate correspondant,
1-[2-(2-chlorophényl)éthyl]-2-(diméthylaminophénylméthyl)cyclohexanol ou le chlorhydrate correspondant,
1-[2-(3-chlorophényl)éthyl]-2-(diméthylaminophénylméthyl)cyclohexanol ou le chlorhydrate correspondant,
1-phénéthyl-2-(phénylpipéridin-1-ylméthyl)cyclohexanol ou le chlorhydrate correspondant,
1-[2-(2-chlorophényl)éthyl]-2-(diméthylaminoo-tolylméthyl)cyclohexanol ou le chlorhydrate correspondant,
1-[2-(3-chlorophényl)éthyl]-2-(diméthylaminoo-tolylméthyl)cyclohexanol ou le chlorhydrate correspondant,
2-[diméthylamino-(2-fluorophényl)méthyl]-1-[2-(4-fluorophényl)éthyl]cyclohexanol ou le chlorhydrate correspondant,
2-(diméthylamino-o-tolylméthyl)-1-[2-(4-fluorophényl)éthyl]cyclohexanol ou le chlorhydrate correspondant,
1-[2-(2-chlorophényl)éthyl]-2-[diméthylamino-(2-fluorophényl)méthyl]cyclohexanol ou le chlorhydrate correspondant,
1-[2-(2-chlorophényl)éthyl]-2-(diméthylaminom-tolylméthyl)cyclohexanol ou le chlorhydrate correspondant,
1-[2-(3-chlorophényl)éthyl]-2-[diméthylamino-(2-fluorophényl)méthyl]cyclohexanol ou le chlorhydrate correspondant,
1-[2-(2-chlorophényl)éthyl]-2-[diméthylamino-(3-fluorophényl)méthyl]cyclohexanol ou le chlorhydrate correspondant,
1-[2-(2-chlorophényl)éthyl]-2-(pyrrolidin-1-yl-o-tolylméthyl)cyclohexanol ou le chlorhydrate correspondant,
1-[2-(2-chlorophényl)éthyl]-2-(morpholin-4-yl-o-tolylméthyl)cyclohexanol ou le chlorhydrate correspondant,
1-[2-(2-chlorophényl)éthyl)-2-(pipéridin-1-yl-o-tolylméthyl)cyclohexanol ou le chlorhydrate correspondant,
1-[2-(3-chlorophényl)éthyl)-2-(diméthylamino-o-tolylméthyl)cyclohexanol ou le chlorhydrate correspondant,
2-[diméthylamino-(2-trifluorométhylphényl)méthyl]-1-phénéthylcyclohexanol ou le chlorhydrate correspondant,
2-[diméthylamino-(2-méthoxyphényl)méthyl]-1-[2-(2-fluorophényl)éthyl]cyclohexanol ou le chlorhydrate correspondant,
2-[diméthylamino-(2-fluorophényl)méthyl]-1-[2-(2-fluorophényl)éthyl]cyclohexanol ou le chlorhydrate correspondant,
2-(diméthylamino-o-tolylméthyl)-1-[2-(2-fluorophényl)éthyl]cyclohexanol ou le chlorhydrate correspondant,
1-[2-(2-fluorophényl)éthyl]-2-(pyrrolidin-1-yl-o-tolylméthyl)cyclohexanol ou le chlorhydrate correspondant,
2-[(2-bromophényl)diméthylaminométhyl]-1-[2-(2-chlorophényl)éthyl]cyclohexanol ou le chlorhydrate correspondant,
2-[(2-chlorophényl)diméthylaminométhyl]-1-[2-(2-chlorophényl)éthyl]cyclohexanol ou le chlorhydrate correspondant,
2-[(2-bromophényl)diméthylaminométhyl]-1-[2-(2-fluorophényl)éthyl]cyclohexanol ou le chlorhydrate correspondant,
2-[(2-chlorophényl)diméthylaminométhyl]-1-(2-o-tolyléthyl)cyclohexanol ou le chlorhydrate correspondant,
2-[(2-chlorophényl)diméthylaminométhyl]-1-(3-phénylpropyl)cyclohexanol ou le chlorhydrate correspondant,
2-(diméthylaminophénylméthyl)-1-(2-o-tolyléthyl)-cyclohexanol ou le chlorhydrate correspondant,
2-[(2-bromophényl)diméthylaminométhyl]-1-(2-o-tolyléthyl)cyclohexanol ou le chlorhydrate correspondant,
2-(diméthylamino-o-tolylméthyl)-1-(2-o-tolyléthyl)cyclohexanol ou le chlorhydrate correspondant,
2-[(2-bromophényl)diméthylaminométhyl]-1-(3-phénylpropyl)cyclohexanol ou le chlorhydrate correspondant,
2-[(2-chloro-4-fluorophényl)diméthylaminométhyl]-1-[2-(2-fluorophényl)éthyl]cyclohexanol ou le chlorhydrate correspondant,
2-[(2-chloro-4-fluorophényl)diméthylaminométhyl]-1-[2-(2-chlorophényl)éthyl]cyclohexanol ou le chlorhydrate correspondant,
2-[diméthylamino-(2-fluorophényl)méthyl]-1-(2-o-tolyléthyl)cyclohexanol ou le chlorhydrate correspondant,
2-[diméthylamino-(2-fluorophényl)méthyl]-1-(3-phénylpropyl)cyclohexanol ou le chlorhydrate correspondant,
2-(diméthylamino-o-tolylméthyl)-1-(3-phénylpropyl)cyclohexanol ou le chlorhydrate correspondant,
1-[2-(2-chlorophényl)éthyl]-2-[diméthylamino-(2-trifluorométhylphényl)méthyl]cyclohexanol ou le chlorhydrate correspondant,
ou
2-[diméthylamino-(2-trifluorométhylphényl)méthyl]-1-[2-(2-fluorophényl)éthyl]cyclohexanol ou le chlorhydrate correspondant.

13. Utilisation selon l'une quelconque des revendications 1 à 12, **caractérisée en ce qu'**on utilise un mélange d'énantiomères d'au moins un composé selon la formule générale I, les énantiomères n'étant pas présents en quantités équimolaires.

14. Utilisation selon la revendication 13, **caractérisée en ce que** l'un des énantiomères est présent dans le mélange d'énantiomères en une proportion relative de 5 à 45 % en masse.
